# EUROPEAN PATENT APPLICATION

(11) **EP 4 541 370 A1**
(43) Date of publication of application: **23.04.2025**
(21) Application number: 23824215.0
(22) Date of filing: 14.06.2023
(51) Int. Cl.: A61K 39/04, A61P 31/06, C07K 14/35

(54) **TUBERCULOSIS VACCINE COMPOSITION CONTAINING IMMUNE-ACTIVE SITE FUSION PROTEIN**

(30) Priority: 17.06.2022 KR 20220074381
(71) Applicant: Myco-Rapha Inc., Daejeon 35015 (KR)
(72) Inventor: KIM, Hwa Jung, Daejeon 34961 (KR); BAEK, Yong Woo, Daejeon 34193 (KR); CHOI, Seung A, Daejeon 34090 (KR); CHOI, Han Gyu, Daejeon 35359 (KR)
(74) Representative: Kraus & Lederer PartGmbB
(86) International application number: PCT/KR2023/008160
(87) International publication number: WO 2023/244001

(57) **Abstract**

The present invention provides a fusion protein vaccine composition by the identification of immune-active domains (sites or segments) of Rv2299c protein, the removal of unnecessary sites or the selection of only necessary portions, and linkage to different immune-active proteins or sites, to effectively activate an immune response in tuberculosis patients, and thus can significantly contribute to the prevention and treatment of tuberculosis.

## Description

### Technical Field

The present disclosure relates to a tuberculosis vaccine composition containing an immune-active site fusion protein.

### Background Art

Tuberculosis (TB) is an infectious disease caused by *Mycobacterium tuberculosis* (Mtb) and is one of the leading causes of death from infectious diseases in human history. In 2019, 1.4 million people worldwide died from tuberculosis, and the mortality rate increased further in 2020 due to limited access to healthcare caused by COVID-19. South Korea is one of the countries with the most serious tuberculosis problems among OECD member states.

To address this tuberculosis issue, the most critical need is a safe and highly effective vaccine. However, the only existing tuberculosis vaccine, *Mycobacterium bovis* Bacillus Calmette-Guérin (BCG), has a reported prevention efficacy ranging from 0% to 80%, depending on the study, and it has been reported to have no preventive effect against latent tuberculosis reactivation or adult tuberculosis. Although BCG has a protective effect against severe tuberculosis in children and is therefore used in many countries, a vaccine significantly more effective than BCG has yet to be developed.

The goal of the World Health Organization's (WHO) End TB Strategy is to reduce the incidence of tuberculosis by 90% and the mortality rate by 95% by 2035, based on 2015 figures. South Korea has also established the "Tuberculosis Eradication 2030 Plan" with the goal of reducing the tuberculosis incidence rate to less than 10 per 100,000 people. To achieve a world without tuberculosis, the development of a tuberculosis vaccine that can serve as a game-changer is essential.

WHO has proposed three objectives for tuberculosis vaccine development: (i) an affordable tuberculosis vaccine for infants and young children with efficacy and safety comparable to BCG, (ii) a safe and effective tuberculosis vaccine for adolescents and adults at an affordable price, and (iii) a therapeutic vaccine to improve tuberculosis treatment efficiency.

Currently, 14 types of tuberculosis vaccines are undergoing clinical trials worldwide. These vaccines can be classified by function as follows: (i) live-attenuated vaccines for replacing the prime vaccine BCG, (ii) BCG booster vaccines applying adjuvants or viral vectors, and (iii) immunotherapeutic vaccines based on cell extracts or killed non-tuberculous mycobacteria to shorten treatment duration or prevent relapse. However, none of these vaccines have yet approached the goals of tuberculosis vaccine development.

The development of live-attenuated vaccines to replace BCG mainly involves recombinant BCG strains or tuberculosis strains with the simultaneous deletion of two genes. These live-attenuated vaccines can induce diverse immune responses as they contain more antigens than subunit vaccines, thereby offering the potential for higher protective efficacy. However, the development of recombinant or gene-deleted strains involves challenges such as removing antibiotic resistance markers and proving safety, resulting in prolonged development times. Even after development, strict quality control is required during the production of vaccine strains. Due to these limitations, the development of BCG replacement vaccines has focused on subunit vaccines using proteins, but these subunit vaccines are primarily developed as BCG boosters rather than direct replacements due to their limitations.

Additionally, the reactivation of latent tuberculosis due to an increase in the aging population, immunosuppressive treatments, and rising HIV infections, along with the emergence of drug-resistant tuberculosis, have made the development of therapeutic vaccines to support treatment a significant issue. Most therapeutic vaccines under development use nontuberculous mycobacteria (NTM) or cell extracts, so a fusion protein-based therapeutic vaccine composed of a few proteins would be ideal.

In conventional technology, Korean Patent No. 1749165 discloses a composition for promoting the maturation of dendritic cells containing Rv2299c or a fusion protein of Rv2299c and ESAT6, and a method for differentiating immature dendritic cells into mature dendritic cells. Additionally, Korean Patent No. 2193304 discloses a composition for a BCG vaccine booster containing the Rv2299c-ESAT6-HspX-RipA fusion protein, and Korean Patent No. 2021-0157659 A discloses a composition for tuberculosis immunotherapy containing the Rv2882c-Rv2005c fusion protein. Korean Patent No. 1452983 also discloses a composition for inducing dendritic cell maturation containing Rv2005c protein of *Mycobacterium tuberculosis* as an active ingredient. However, the problem with such fusion proteins is that the increase in molecular weight makes standardization of the purification process difficult. Therefore, there is a need to develop a vaccine composition with reduced molecular weight while maximizing immunological activity.

### Disclosure of Invention

### Technical Problem

The present disclosure aims to provide a tuberculosis vaccine composition containing an immune-active site fusion protein.

Also, the present disclosure is to provide a tuberculosis prophylactic vaccine or a tuberculosis therapeutic vaccine containing the immune-active site fusion protein.

### Solution to Problem

The present disclosure provides a tuberculosis vaccine composition containing a fusion protein of an immune-active site of Rv2299c, and ESAT6.

The immune-active site of Rv2299c may consist of one amino acid sequence selected from the group consisting of SEQ ID NO: 1 to SEQ ID NO: 5. Furthermore, the immune-active site of Rv2299c may be domain 2 (D2) consisting of the amino acid sequence of SEQ ID NO: 3, domain 3 (D3) consisting of the amino acid sequence of SEQ ID NO: 4, or a combination of domain 2 and domain 3 (D2D3) consisting of the amino acid sequence of SEQ ID NO: 5.

The present disclosure provides a tuberculosis vaccine composition containing a fusion protein of an immune-active site of Rv2299c, and ESAT6.

The immune-active site of Rv2299c may consist of one amino acid sequence selected from the group consisting of SEQ ID NO: 1 to SEQ ID NO: 5. Furthermore, the immune-active site of Rv2299c may be domain 2 (D2) consisting of the amino acid sequence of SEQ ID NO: 3, domain 3 (D3) consisting of the amino acid sequence of SEQ ID NO: 4, or a combination of domain 2 and domain 3 (D2D3) consisting of the amino acid sequence of SEQ ID NO: 5.

The present disclosure provides a tuberculosis vaccine composition, wherein the fusion protein of the immune-active site of Rv2299c and ESAT6 has a Bacillus Calmette-Guérin-Cell Wall Skeleton (BCG-CWS) conjugated to the terminus thereof.

The tuberculosis vaccine composition with any formulation provided herein may be used as either a prophylactic or therapeutic vaccine for tuberculosis.

According to the present disclosure, the fusion protein of the immune-active site of Rv2299c and ESAT6 may further include at least one *Mycobacterium tuberculosis*-derived antigen selected from the group consisting of RpfE, Rv3463, Rv2005c, Rv2882c, Rv2145c, Rv1605, Rv2220, and Rv0869c, or an immune-active site thereof. In this regard, the *Mycobacterium tuberculosis-*derived antigen or an immune-active site thereof may be an antigen including at least one amino acid sequence selected from the group consisting of SEQ ID NOS: 1 to 22, or an immune-active site thereof.

More specifically, the present disclosure provides a tuberculosis vaccine composition including a fusion protein of an immune-active site of Rv9922c and ESAT6 plus a *Mycobacterium tuberculosis*-derived antigen or an immune-active site thereof conjugated to the terminal end of the fusion protein, wherein the *Mycobacterium tuberculosis*-derived antigen is selected from the group consisting of RpfED1 including the amino acid sequence of SEQ ID NO: 8, Rv3463 including the amino acid sequence of SEQ ID NO: 19, Rv2005cD3-Rv3463 including a fused amino acid sequence in which the amino acid sequences of SEQ ID NOS: 18 and 19 are sequentially linked, Rv2882cD2-Rv2005cD3-Rv3463D2 including a fused amino acid sequence in which the amino acid sequences of SEQ ID NOS: 12, 18, and 21 are sequentially linked, Rv2005cD3-Rv1605-Rv3463D2 including a fused amino acid sequence in which the amino acid sequences of the amino acid sequences of SEQ ID NOS: 18, 22, and 21 are sequentially linked, Rv3463D2-Rv2882cD2 including a fused amino acid sequence in which the amino acid sequences of the amino acid sequences of SEQ ID NOS: 21 and 12 are sequentially linked, Rv3463D2-Rv2882cD2-Rv2145cD2 including a fused amino acid sequence in which the amino acid sequences of the amino acid sequences of SEQ ID NOS: 21, 12, and 15 are sequentially linked, Rv3463D2-Rv2882cD2-RpfED1 including a fused amino acid sequence in which the amino acid sequences of the amino acid sequences of SEQ ID NOS: 21, 12, and 8 are sequentially linked, Rv3463D2-Rv2882cD2-RpfED1-Rv2145cD2 including a fused amino acid sequence in which the amino acid sequences of SEQ ID NOS: 21, 12, 8, and 15 are sequentially linked, Rv3463D2-Rv2005cD3 including a fused amino acid sequence in which the amino acid sequences of the amino acid sequences of SEQ ID NOS: 21 and 18 are sequentially linked, and Rv3463D2-Rv2005cD3-RpfED1 including a fused amino acid sequence in which the amino acid sequences of the amino acid sequences of SEQ ID NOS: 21, 18, and 8 are sequentially linked.

In the present disclosure, the fusion protein represented by Rv2299cD2D3-ESAT6 refers to a polypeptide in which the amino acid sequences of SEQ ID NOS: 5 and 6 are sequentially linked to each other, with one terminus of Rv2299cD2D3 being connected to one terminus of ESAT6. The production of the Rv2299cD2D3-ESAT6 fusion protein is achieved using the recombinant protein production method utilizing pET-22b(+)_Rv2299cD2D3-ESAT6 as described in Example 1.2.

As used herein, the term "fusion protein containing two or more *Mycobacterium tuberculosis-*derived antigens or immune-active sites thereof" refers to a polypeptide in which the amino acid sequences of the respective antigens or immune-active sites are sequentially linked to each other, with or without a linker.

As used herein, the term "prophylactic vaccine" in the present disclosure refers to a vaccine to be administered prior to pathogen exposure, enabling the immune system to prevent disease by activating upon pathogen invasion. The term "therapeutic vaccine" refers to a vaccine designed to treat patients who are already infected, while typically also possessing prophylactic functions. Tuberculosis prophylactic vaccines include priming vaccines and booster vaccines. Priming vaccines are administered in infancy and aim to provide immunity against initial exposure to *M. tuberculosis.* Booster vaccines are administered to adolescents or adults to enhance the immune response after priming or latent tuberculosis infection. Therapeutic vaccines are administered over a short period in conjunction with direct drug treatment.

The fusion protein containing *Mycobacterium tuberculosis*-derived antigens or immune-active sites thereof according to the present disclosure, may increase immunological activity by further conjugating the BCG Cell Wall Skeleton (BCG-CWS) to the terminal end thereof.

Furthermore, the present disclosure provides a tuberculosis vaccine composition including any one fusion protein selected from the group consisting of Rv2882c-Rv2005c-Rv3463 including a fused amino acid sequence in which the amino acid sequences of SEQ ID NOS: 10, 16, and 19 are sequentially linked, Rv2882c-Rv2005cD3-ESAT6-Rv3463 including a fused amino acid sequence in which the amino acid sequences of SEQ ID NOS: 10, 16, 6, and 19 are sequentially linked, Rv2220-Rv2882cD2-Rv2005cD3-ESAT6-Rv3463D2, Rv0869c-Rv2882cD2-Rv2005cD3-ESAT6-Rv3463D2 including a fused amino acid sequence in which the amino acid sequences of SEQ ID NOS: 23, 12, 15, 6, and 21 are sequentially linked, Rv1605-Rv2882cD2-Rv2005cD3-ESAT6-Rv3463D2 including a fused amino acid sequence in which the amino acid sequences of SEQ ID NOS: 22, 12, 18, 6, and 21 are sequentially linked, Rv2220-ESAT6-Rv3463 including a fused amino acid sequence in which the amino acid sequences of SEQ ID NOS: 23, 6, 19, 6, and 21 are sequentially linked, Rv0869c-ESAT6-Rv2005cD3-Rv3463 including a fused amino acid sequence in which the amino acid sequences of SEQ ID NOS: 24, 6, 18, and 19 are sequentially linked, and Rv1605-ESAT6-Rv2005cD3-Rv3463 including a fused amino acid sequence in which the amino acid sequences of SEQ ID NOS: 22, 6, 18, and 19 are sequentially linked.

The fusion protein may have the BCG Cell Wall Skeleton (BCG-CWS) attached to the terminal end thereof to increase immunological activity. The tuberculosis vaccine composition may be used as either a prophylactic or therapeutic vaccine.

The fusion proteins included in the tuberculosis vaccine composition of the present disclosure can be produced by known methods in the art. For example, they may be produced through recombinant or synthetic methods using an automated polypeptide synthesizer and can be mass-produced by inserting into a vector and transforming *Escherichia coli* or other organisms. The fusion protein or antigen may include an appropriate purification tag (or affinity tag) to allow purification from unpurified biological sources (e.g., recombinant expression systems). Purification tags include His-tags, chitin-binding proteins (CBP), maltose-binding proteins (MBP), and glutathione-S-transferase (GST), but are not limited thereto.

### Advantageous Effects of Invention

The present disclosure provides a fusion protein vaccine composition including a fusion protein prepared by identifying an immune-active domain (site or segment) of Rv2299c protein, removing unnecessary portions or selectively retaining only a required part, and conjugating the required part to another immune-active protein or site, and the fusion protein vaccine composition effectively activates the immune response in tuberculosis patients, making a significant contribution to the prevention and treatment of tuberculosis.

### Brief Description of Drawings

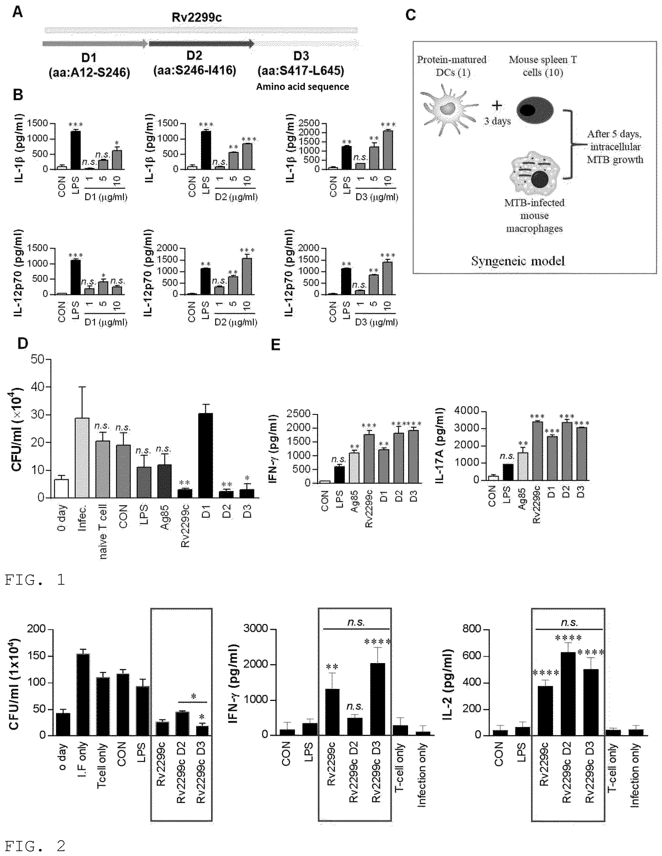

FIG. 1 shows Rv2299c domain constructs and the degrees of immune cell activation for each domain: (A) Rv2299c domain construction; (B) confirmation of dendritic cell activation ability of the produced Rv2299c domains; (C) schematic view of the method for measuring anti-tuberculosis activity; (D) measurement of anti-tuberculosis activity of Rv2299c domains, and (E) measurement of cytokines produced under condition D using ELISA to evaluate T-cell activation by Rv2299c domains.
FIG. 2 shows graphs comparing the anti-tuberculosis activity and T-cell activation (IFN-γ and IL-2 production) effects of the first-selected Rv2299c domains D2 and D3.

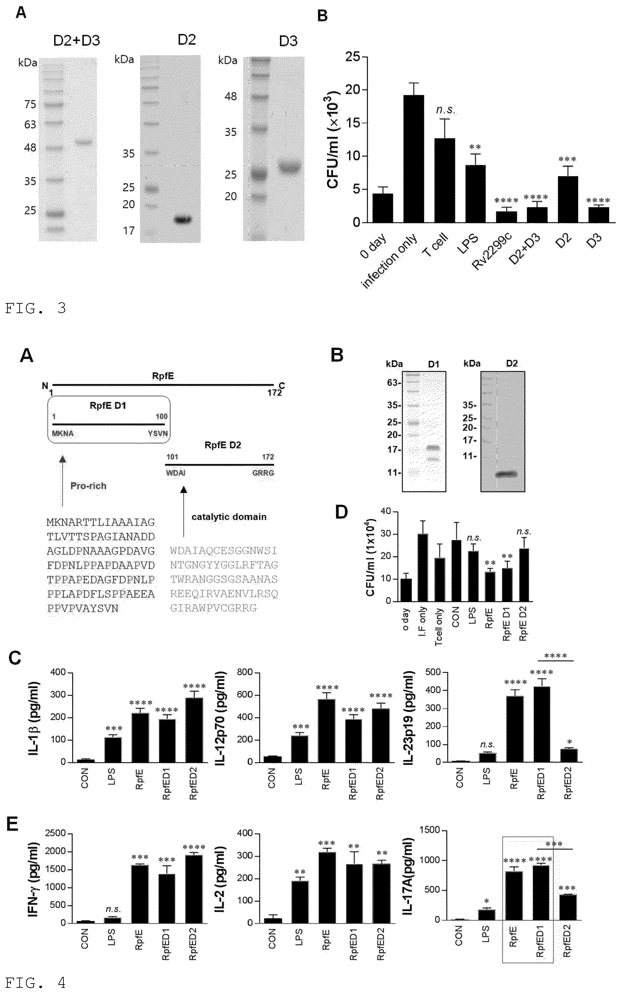

FIG. 3 shows the construction of the Rv2299c D2, D3, and D2+D3 fusion domains and their anti-tuberculosis activity: (A) purification of Rv2299c domains and fusion domains; and (B) a graph showing tuberculosis bacteria counts after dendritic cells were activated with each stimulant for 24 hours and co-cultured with T-cells and then with macrophages infected with *Mycobacterium tuberculosis* for 72 hours.
FIG. 4 shows RpfE domain constructs and the evaluation of the domain constructs for anti-tuberculosis activity through T-cell activation: (A, B) RpfE domain construction; (C) graphs analyzing dendritic cell activation using ELISA after dendritic cells were activated with each stimulant for 24 hours; (D) a graph measuring tuberculosis bacteria counts after dendritic cells were activated with each stimulant for 24 hours and co-cultured with T-cells and then with macrophages infected with *Mycobacterium tuberculosis* for 72 hours; and (E) measurement of cytokines produced by T-cells under condition D.

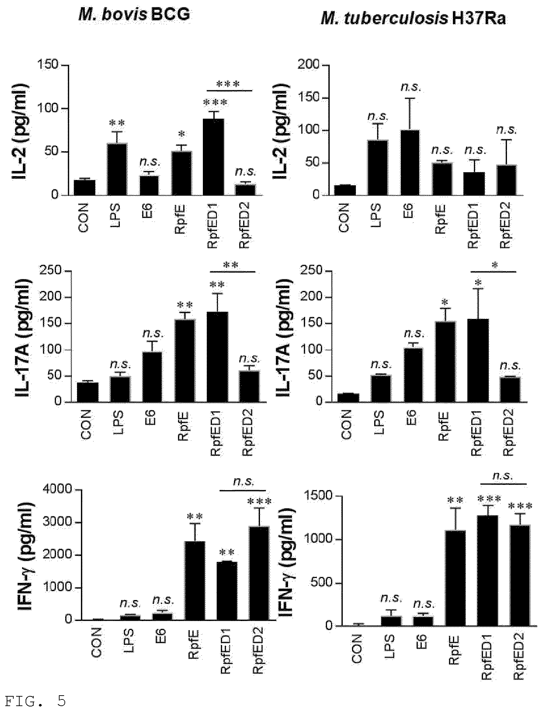

FIG. 5 shows graphs of the T-cell differentiation by the RpfE, D1, and D2 domains evaluated by measuring through ELISA the levels of IFN-γ, IL-17, and IL-2 produced after dendritic cells activated with each stimulant for 24 hours were co-cultured with T-cells from the spleen of BCG- or Mtb H37Ra-infected mice for 72 hours.

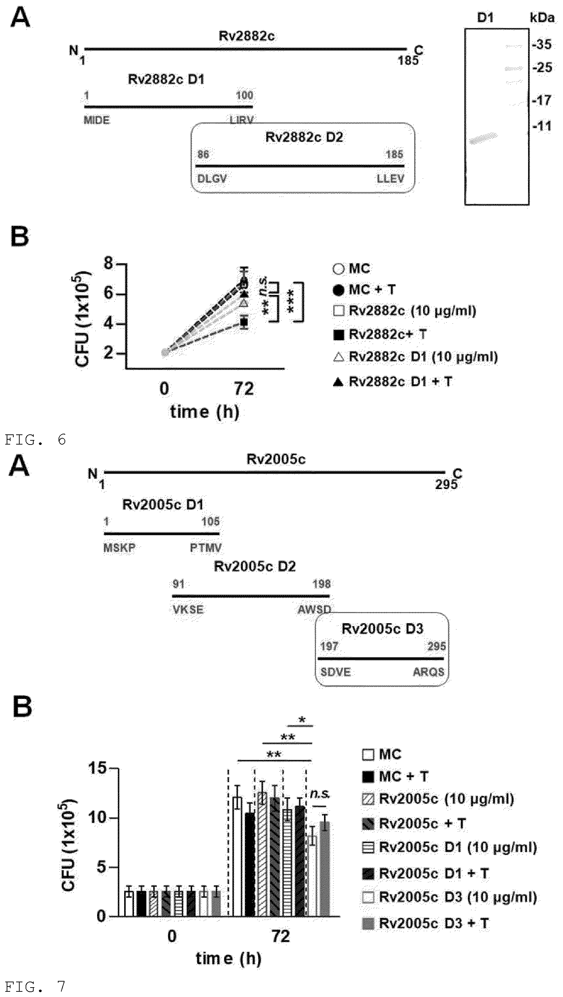

FIG. 6 shows the effects of Rv2882c, D1, and D2 domains on *M. tuberculosis* growth under T-cell co-culture conditions: (A) as Rv2882c domain proteins, an N-terminal part (1-100; Rv2882c D1) and a C-terminal part (86-185; Rv2882c D2) were set and subjected to expression in E. coli, but only Rv2882c D1 expression was confirmed by SDS-PAGE; and (B) After infection of BMDMs with *Mtb* (MOI:1) for 4 hours, the total Rv2882c and Rv2882c D1 were treated for 24 hours and co-cultured with splenocytes for 72 hours to confirm intracellular *Mtb* growth.
FIG. 7 shows the effects of Rv2005c, D1, and D3 domains on *Mtb* growth: (A) as Rv2005c protein domains, D1 (1-105), D2 (91-198), and D3 (197-294) parts were set and subjected to expression in E. coli, but only Rv2005c D1 and D3 were confirmed to be expressed; and (B) after infection with *Mtb* (MOI:1) for 4 hours, BMDMs were incubated with full-length Rv2005c, Rv2005c D1, and Rv2005c D3 for 24 hours, and co-cultured with splenocytes at a ratio of 1:5-10 for 72 hours to confirm intracellular *Mtb* growth.

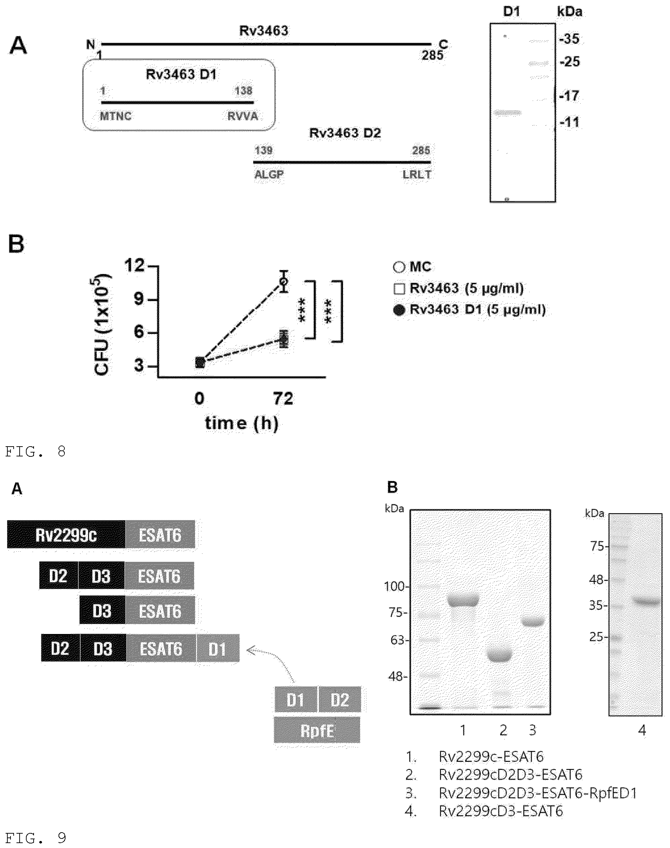

FIG. 8 shows that the N-terminal region of Rv3463 is a domain that regulates *Mtb* growth: (A) Rv3463 protein was divided into an N-terminal part (1-138; Rv3463 D1) and a C-terminal part (139-285; Rv3463 D2). Individual truncated proteins were subjected to expression in E. coli, but only Rv3463 D2 expression was confirmed, and the expressed Rv3463 D2 part was purified and analyzed by SDS-PAGE; and (B) BMDMs were infected with Mtb (MOI:1) for 4 hours and incubated with full-length Rv3463 and Rv3463 D2 for 72 hours. Intracellular Mtb growth was measured after 72 hours.
FIG. 9 shows (A) a schematic diagram of the fusion protein composition of ESAT6 and each domain, and (B) a purification image of each fusion protein.

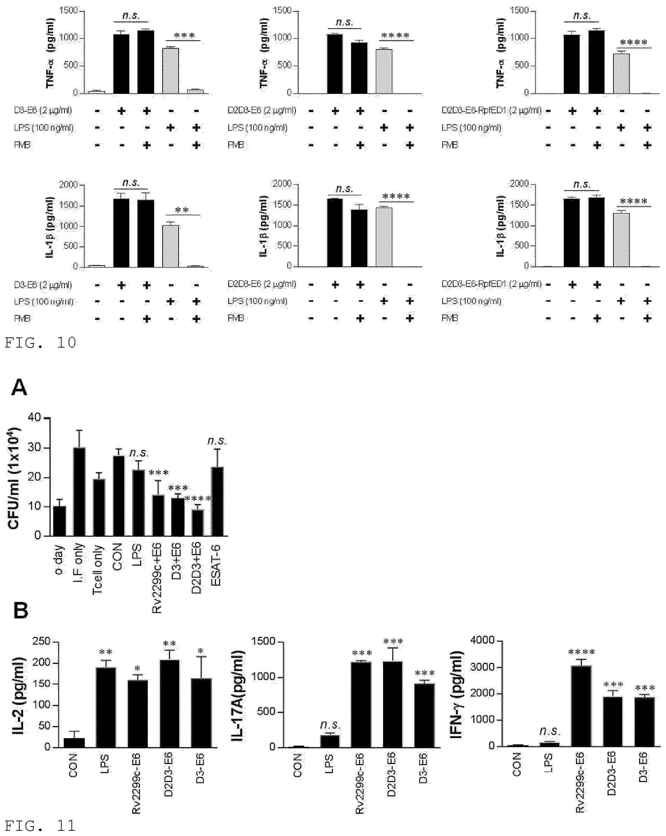

FIG. 10 shows the measurements of LPS contamination in the purified fusion proteins: after pre-treatment of dendritic cells with the LPS inhibitor PMB, they were activated for 24 hours with each stimulant, and the activation of dendritic cells (TNF-α, IL-1β) was analyzed using ELISA.
FIG. 11 shows the evaluation of anti-tuberculosis activity and T-cell activation of the purified fusion proteins: (A) after dendritic cells were activated for 24 hours with each stimulant and co-cultured for 72 hours with T-cells and then for 72 hours with T-cells activated by macrophages infected with tuberculosis bacteria, the tuberculosis bacteria were counted; and (B) after dendritic cells were activated for 24 hours with each stimulant and then co-cultured for 72 hours , T-cells were co-cultured for 72 hours, and T-cell activation (IL-2, IL-17, INF-γ) was analyzed using ELISA.

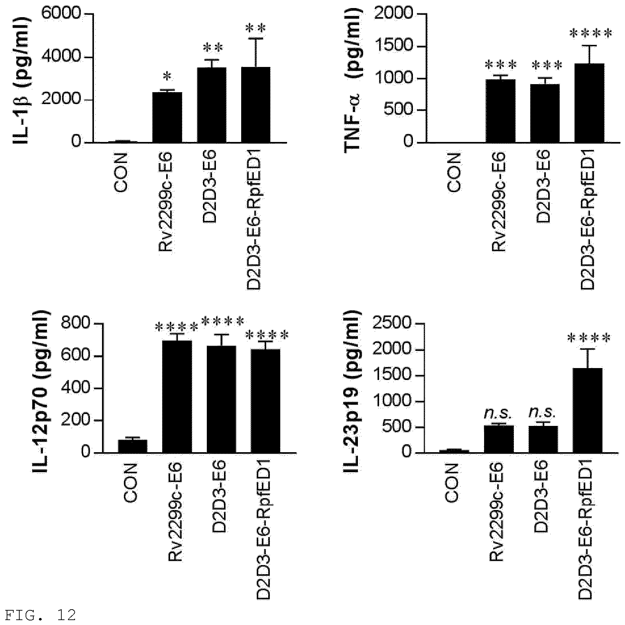

FIG. 12 shows the evaluation of dendritic cell activation by the purified fusion proteins: after being activated for 24 hours with each stimulant, dendritic cells were analyzed for activity (IL-1β, TNF-α, IL-12, IL-23) using ELISA.

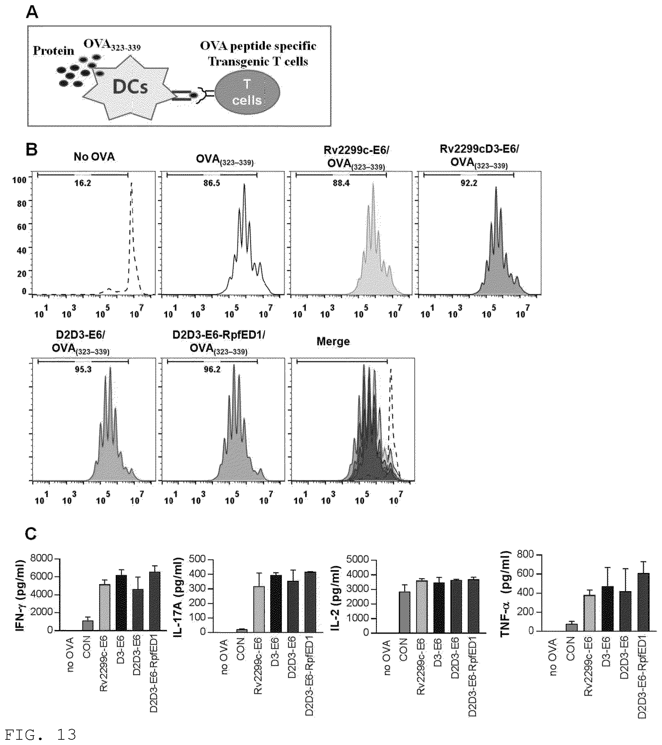

FIG. 13 shows the evaluation of the proliferation ability of naïve T-cells activated by dendritic cells activated with the fusion proteins: (A) a schematic diagram of the method for evaluating naive T-cell proliferation; and (B) dendritic cells were treated with the constructed fusion proteins and ovalbumin (OVA) peptides for 24 hours and the OVA peptide-specific T-cells were pre-treated with CFSE and co-cultured with activated dendritic cells for 24 hours, followed by FACS analysis, and (C) analysis of T-cell activation (INF-γ, IL-17, IL-2, TNF-α).

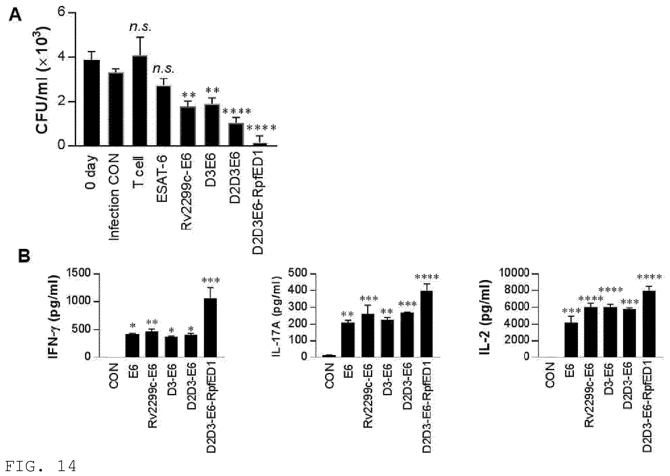

FIG. 14 shows the evaluation of anti-tuberculosis activity of T-cells activated by fusion proteins: (A) dendritic cells were activated for 24 hours with each stimulant, followed by co-culturing T-cells and tuberculosis-infected macrophages for 72 hours. The tuberculosis bacteria count was then measured. (B) After 24 hours of stimulation with dendritic cells, T-cells were co-cultured for 72 hours, and T-cell activation (INF-γ, IL-17, IL-2) was analyzed using ELISA.

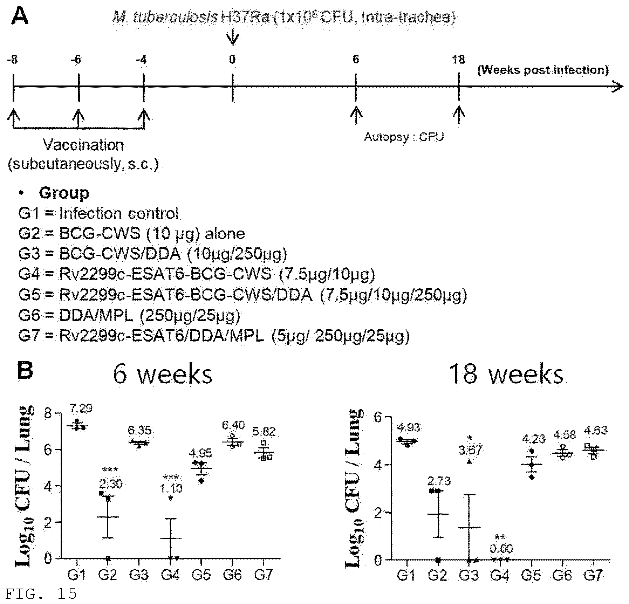

FIG. 15 shows the evaluation of the efficacy of the Rv2299c-ESAT6 and BCG-CWS fusion vaccine in a non-pathogenic tuberculosis model in mice. (A) Schematic diagram of the experimental design for evaluating vaccine efficacy. (B) Bacterial load in the lungs of each group 6 or 18 weeks after tuberculosis infection.

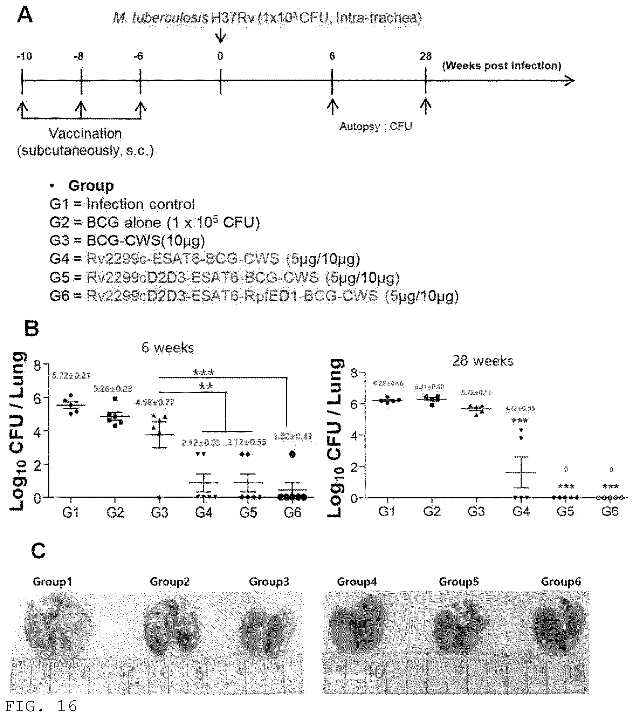

FIG. 16 shows the evaluation of the efficacy of the Rv2299cD2D3-BCG-CWS fusion vaccine in a pathogenic tuberculosis model in mice. (A) Schematic diagram of the experimental design for evaluating vaccine efficacy. (B) Bacterial load in the lungs of each group 6 or 28 weeks after infection with pathogenic tuberculosis. (C) Pathological lesions in the lungs of each group 28 weeks after tuberculosis infection.

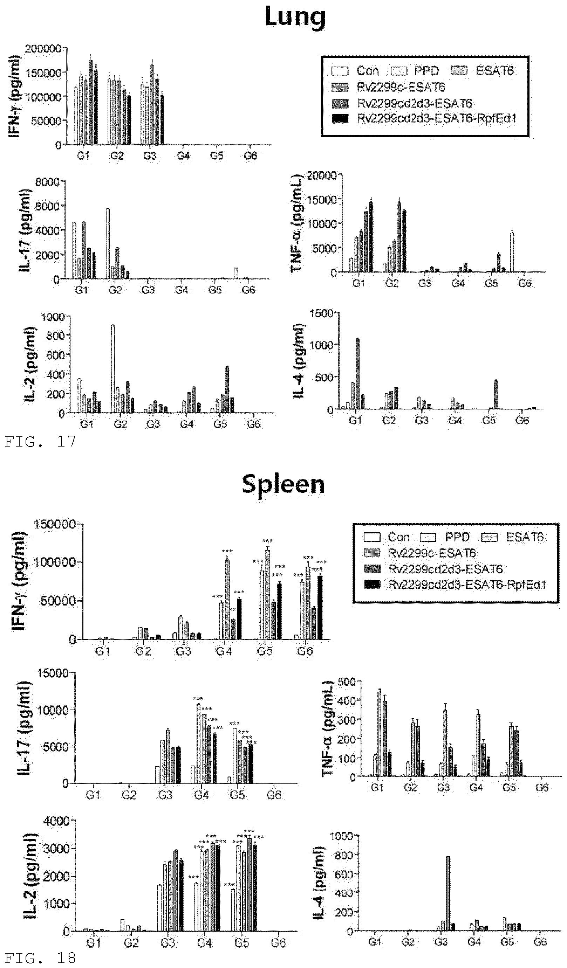

FIG. 17 shows the analysis of antigen-specific cytokines in the lungs of mice 6 weeks after infection with pathogenic tuberculosis.
FIG. 18 shows the analysis of antigen-specific cytokines in the spleen of mice 6 weeks after infection with pathogenic tuberculosis.

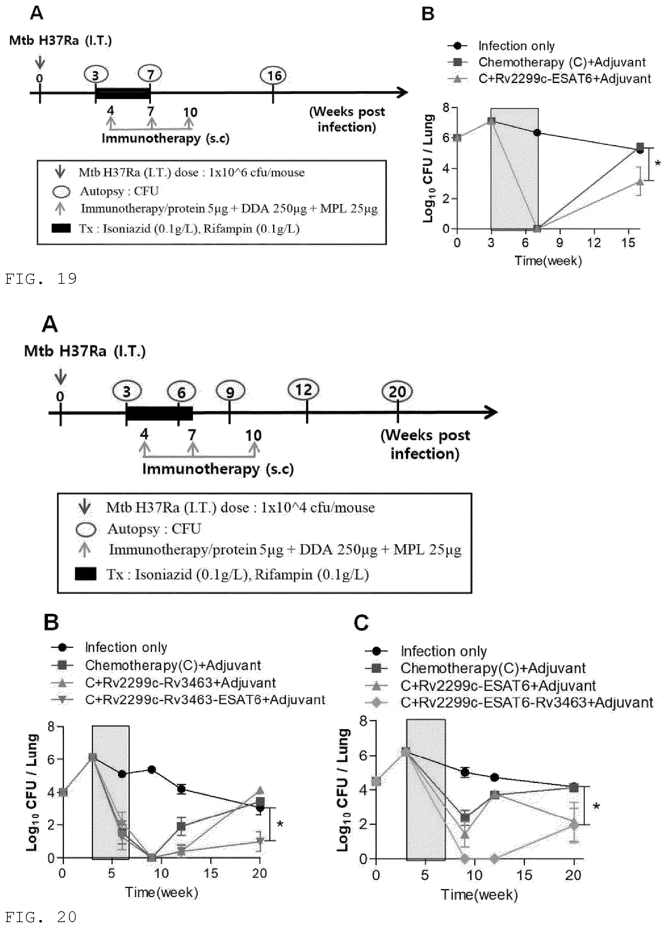

FIG. 19 shows the evaluation of the therapeutic vaccine efficacy of Rv2299c-ESAT6 using a latent infection model. (A) Strategy schedule for efficacy evaluation using the Mtb H37Ra latent infection model. (B) Bacterial load in the lungs of each group according to the evaluation schedule after tuberculosis infection.
FIG. 20 shows the evaluation of the therapeutic vaccine efficacy of Rv2299c-ESAT6-Rv3463 using a latent infection model. (A) Strategy schedule for efficacy evaluation using the Mtb H37Ra latent infection model. (B), (C) Bacterial load in the lungs of each group based on repeated evaluations after tuberculosis infection.

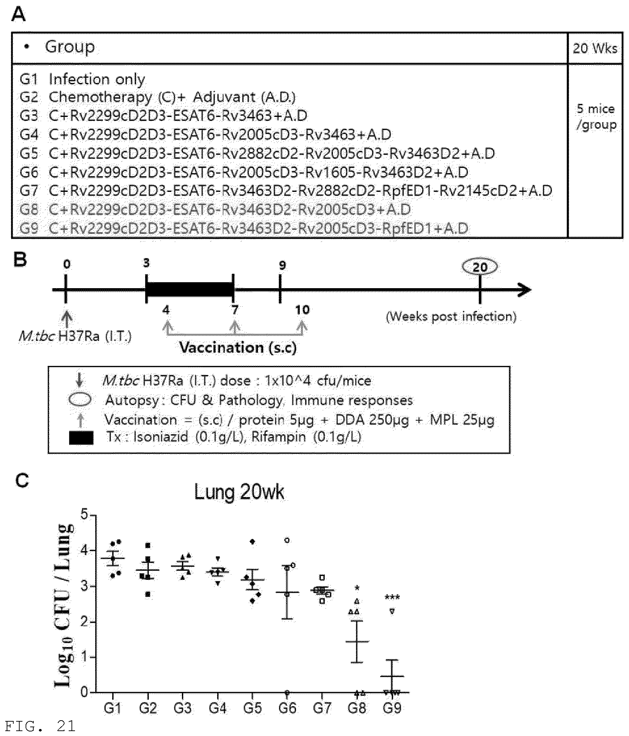

FIG. 21 shows the evaluation for therapeutic vaccine efficacy of Rv2299cD2D3-ESAT6 backbone-structured vaccine candidates: (A) groups of Rv2299cD2D3-ESAT6 backbone vaccine candidates; (B) the efficacy evaluation strategy schedule using the Mtb H37Ra latent infection model; and (C) bacterial burden in the lungs of each candidate group 20 weeks after tuberculosis infection.

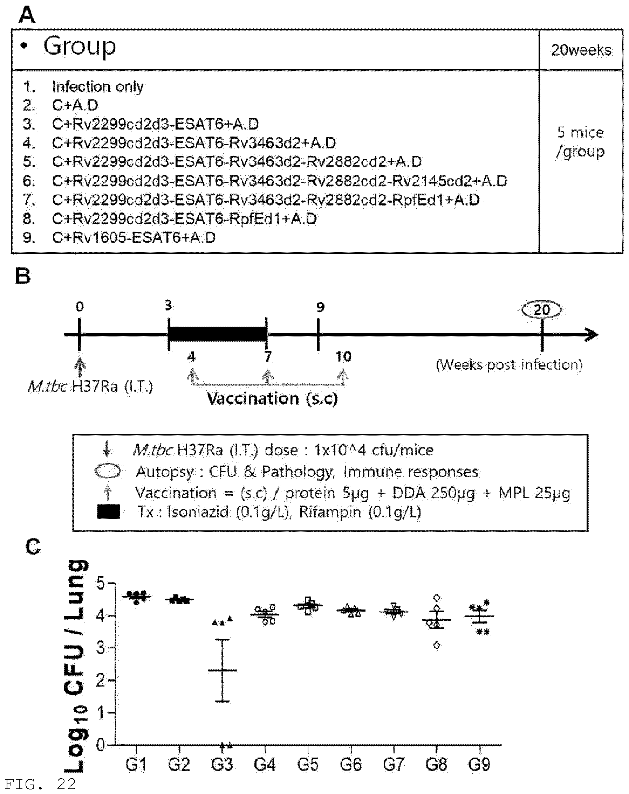

FIG. 22 shows the evaluation for therapeutic vaccine efficacy of Rv2299cD2D3-ESAT6-Rv3463 backbone-structured vaccine candidates: (A) groups of Rv2299cD2D3-ESAT6-Rv3463 backbone-structured vaccine candidates; (B) the efficacy evaluation strategy schedule using the Mtb H37Ra latent infection model; and (C) bacterial burden in the lungs of each candidate group 20 weeks after tuberculosis infection.

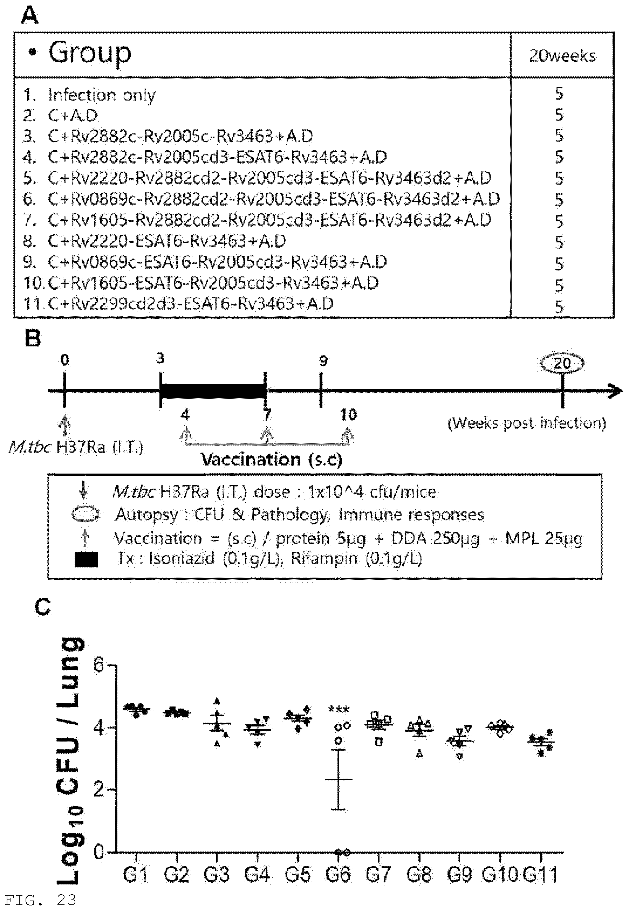

FIG. 23 shows the evaluation for therapeutic vaccine efficacy of the fusion protein with a new antigen and ESAT6-conjugated backbone. (A) groups of vaccine candidates with a new antigen and ESAT6-conjugated backbone structure; (B) efficacy evaluation strategy schedule using the Mtb H37Ra latent infection model; and (C) bacterial burden in the lungs of each candidate group 20 weeks after tuberculosis infection.

### Best Mode for Carrying out the Invention

The inventors have conducted research to develop fusion protein-based vaccines, which can serve as BCG replacements, BCG boosters, or therapeutic vaccines, with excellent protective effects and significant advantages in terms of safety. In particular, the inventors have worked to develop a vaccine with superior protective efficacy that overcomes the shortcomings of the tuberculosis vaccine using Rv2299c-ESAT6, as described in the related art, Korean Patent No. 1749165. When fusing highly immune-active proteins, even if the vaccine has good efficacy, it has been challenging to standardize the purification methods during the commercialization process. Specifically, fusion proteins tend to have increased molecular weight, making them difficult to purify, and prone to denaturation during the purification process. During the course of overcoming these issues, the inventors confirmed that protein-based vaccines are more advantageous for standardizing purification processes when they have smaller molecular weights and that combining other proteins thereto can yield higher efficiency.

Leading to the present disclosure, research has been made, based on the assumption that when constructed by identifying an immune-active domain (site, or segment) within the Rv2299c protein and removing unnecessary portions or selectively retaining only the required parts, a fusion protein could be used as a vaccine with superior efficacy, compared to existing fusion proteins.

Hereinafter, preferable embodiments of the present disclosure will be described in detail. However, the present disclosure is not limited to the embodiments described herein but may be embodied in other forms. Rather, these embodiments are provided so that this disclosure will be thorough and complete, and will fully convey the scope of the invention to those skilled in the art.

### <Experimental Materials and Methods>

### 1. Cloning, Production, and Purification for Recombinant Protein Production

### 1.1 Cloning for Production of Recombinant Proteins by Protein Domain

To produce each recombinant protein required for experiments, gene amplification was made by PCR using the primers listed in Table 1, below, with genomic DNA from *Mycobacterium tuberculosis* H37Rv (ATCC 27294) serving as a template. The PCR products thus achieved were inserted into the pET-22b(+) vector (Novagen, Madison, WI, USA) using the inserted restriction enzyme sequences, and the generated plasmids were confirmed by sequence analysis.

**TABLE 1**

| **Primer** | **Gene Sequence** | **Restriction enzyme sequence inserted** |
|---|---|---|
| Rv2299c D1-Foward | CATATGAACGCCCATGTCGAGCAGTTG | NdeI |
| Rv2299c D1-Reverse | AAGCTTCTTCATCGAGTTGAGGGT | HindIII |
| Rv2299c D2-Foward | CATATGAACCTGGTCAAGAAATAC | NdeI |
| Rv2299c D2-Reverse | AAGCTTGGCGTAGAAGATCTGTTG | HindIII |
| Rv2299c D3-Foward | CATATGGAGGGACTGCTGTCAGAC | NdeI |
| Rv2299c D3-Reverse | AAGCTTCAAGGTACGCGCGAGACGTTC | HindIII |
| RpfE D1-Foward | CATATGGACGACGCGGGCTTGGACCCA | NdeI |
| RpfE D1-Reverse | AAGCTTCACGCTGTAGGCCACGGGCAC | HinIII |
| RpfE D2-Foward | CATATGGTGAACTGGGACGCGATCGCGCAG | NdeI |
| RpfE D2-Reverse | AAGCTTGCCGCGGCGGCCGCAGACCGG | HindIII |
| Rv2882c D1-Foward | 5'-CATATGATTGATGAGGCTCTCTTCGAC-3' | *Nde*I |
| Rv2882c D1-Reverse | 5'-AAGCTTGTTGGTGGGATTCACTCCAAG-3' | *Hind*III |
| Rv2882c D2-Foward | 5'-CATATGGACGGCGCCCTTATTCGCGTG-3' | *Nde*I |
| Rv2882c D2-Reverse | 5'-AAGCTTGACCTCCAGCAGCTCGCCTTC-3' | *Hind*III |
| Rv2005c D1-Foward | 5'-CATATGTCTAAACCCCGCAAGCAGCAC-3' | *Nde*I |
| Rv2005c D1-Reverse | 5'-AAGCTTCTCGTTGGAGATTTCAACCAT-3' | *Hind*III |
| Rv2005c D2-Foward | 5'-CATATGGCAGAGATGGTGGTGTTGGGC-3' | *Nde*I |
| Rv2005c D2-Reverse | 5'-AAGCTTCGCGTGCACGGCGATCAGTTC-3' | *Hind*III |
| Rv2005c D3-Foward | 5'-CATATGTGGAGTGACGTCGAAGTGGTG-3' | *Nde*I |
| Rv2005c D3-Reverse | 5'-AAGCTTCGACTGCCGTGCCACGATCAC-3' | *Hind*III |
| Rv3463 D1-Foward | 5'-CATATGACCAATTGTGCCGCCGGCAAA-3' | *Nde*I |
| Rv3463 D1-Reverse | 5'-AAGCTTGGCCACCACCCGGCGGTTGGC-3' | *Hind*III |
| Rv3463 D2-Foward | 5'-CATATGGCACTGGGCCCCCGGGTCCTG-3' | *Nde*I |
| Rv3463 D2-Reverse | 5'-AAGCTTAGTCAGTCGGAGCGGCTTCGC-3' | *Hind*III |

### 1.2 Cloning for Production of Fusion Proteins (1) pET-22b(+)_Rv2299cD2D3-ESAT6

Genomic DNA from *Mycobacterium tuberculosis* (Mtb) H37Rv (ATCC 27294) was used as a template to obtain Rv2299c DNA and ESAT6 DNA by PCR. Using overlapping PCR, the Rv2299c D2D3-ESAT6 DNA fragment with the restriction enzyme sites NdeI and HindIII inserted respectively at the 5' and 3' ends thereof was constructed, followed by insertion into the pET22b vector. The primers used are as follows.
D2D3-ESAT6_NdeI_F : AAGGAGATATACATATGtcgatgaaggcgctgtgg
D2D3-ESAT6_HindIII_R : GTGCGGCCGCAAGCTTtgcgaacatcccagtgacg
D2D3-ESAT6_intP : aagaaggtgctgtccacg

### (2) pET-22b(+)_Rv2299cD3-ESAT6

From Rv2299c and ESAT6 DNA obtained in the same manner as described above, an Rv2299c D3-ESAT6 DNA fragment with the restriction enzyme sites NdeI and HindIII inserted respectively at the 5' and 3' ends thereof was constructed using an overlapping PCR method, followed by insertion into the pET22b vector. The primers used are as follows.
D3-ESAT6_NdeI_F : AAGGAGATATACATATGctcggtatttcttcgtttgtctc
D3-ESAT6_HindIII_R : GTGCGGCCGCAAGCTTtgcgaacatcccagtgac

### (3) pET-22b(+)_Rv2299cD2D3-ESAT6-RpfE D1

While RpfE DNA and Rv2299c D2D3-ESAT6 DNA obtained in the same manner as described above served as a template, an Rv2299c D2D3-ESAT6-RpfE D1 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c(D2D3)+ESAT6_NdeI_F :
AAGGAGATATACATATGTCGATGAAGGCGCT
Rv2299c(D2D3)+ESAT6_R : TGCGAACATCCCAGTGAC
RpfEdomain1_F : GTCACTGGGATGTTCGCAGCCGACGACGCGGGCTTG
RpfEdomain1_XhoI_R :
   GGTGGTGGTGGTGGTGCTCGAGGTTGTAGGCCACGGGCAC

### (4) pET-22b(+)_Rv2299c-Rv3463-ESAT6

While Rv2299c, Rv3463, and ESAT6 DNA obtained in the same manner as described above served as a template, an Rv2299c-Rv3463-ESAT6 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c-Nde-F : CATATGAACGCCCATGTCGAGCAGTTG
Rv2299c-EcoR-R : GAATTCGGCAAGGTACGCGCGAGACGTTC
Rv3463-EcoR-F : GAATTCGATGACCAATTGTGCCGCC
Rv3463-Hind-R : AAGCTTAGTCAGTCGGAGCGGCTT
ESAT6-Hind-F : AAGCTTATGACAGAGCAGCAGTGGAAT
ESAT6-Xho-R : CTCGAGTGCGAACATCCCAGTGACGTT

### (5) pET-22b(+)_Rv2299c-ESAT6-Rv3463

While Rv2299c, ESAT6, and Rv3463 DNA obtained in the same manner as described above served as a template, an Rv2299c-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c-Nde-F : CATATGAACGCCCATGTCGAGCAGTTG
Rv2299c-EcoR-R : GAATTCGGCAAGGTACGCGCGAGACGTTC
ESAT6-EcoR-F : GAATTCGATGACAGAGCAGCAGTGGAAT
ESAT6-Hind-R : AAGCTTTGCGAACATCCCAGTGACGTT
Rv3463-Hind-F : AAGCTTATGACCAATTGTGCCGCC
Rv3463-Not-R : GCGGCCGCAGTCAGTCGGAGCGGCTT

### (6) pET-22b(+)_Rv2299cD2D3-ESAT6-Rv3463

While Rv2299c D2D3, ESAT6, and Rv3463 DNA obtained in the same manner as described above served as a template, an Rv2299c D2D3-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c_D2D3_NdeI_F : AAGGAGATATACATATGTCGATGAAGGCGCTG
Rv2299c_D2D3_R : CAAGGTACGCGCGAGACG
ESAT-6_F :
   CGTCTCGCGCGTACCTTGGAATTCATGACAGAGCAGCAGTGG
ESAT-6_R : TGCGAACATCCCAGTGAC
Rv3463_F :
   GTCACTGGGATGTTCGCAAAGCTTATGACCAATTGTGCCGCC
Rv3463_NotI_R : TGCTCGAGTGCGGCCGCAGTCAGTCGGAGCGGCTT

### (7) pET-22b(+)_Rv2299cD2D3-ESAT6-Rv2005cD3-Rv3463

While Rv2299c D2D3, ESAT6, Rv2005cD3, and Rv3463 DNA obtained in the same manner as described above served as a template, an Rv2299c D2D3-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c_D2D3_NdeI_F : AAGGAGATATACATATGTCGATGAAGGCGCTG
Rv2299c_D2D3_R : CAAGGTACGCGCGAGACG
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_R : CGACTGCCGTGCCACGAT
Rv3463(NheI)_F : GCACGGCAGTCGGCTAGCATGACCAATTGTGCCGCC
Rv3463_NotI_R : TGCTCGAGTGCGGCCGCAGTCAGTCGGAGCGGCTT

### (8) pET-22b(+)_Rv2299cD2D3-ESAT6-Rv2882cD2-Rv2005cD3-Rv3463D2

While Rv2299c D2D3, ESAT6, Rv2882cD2, Rv2005cD3, and Rv3463 D2 DNA obtained in the same manner as described above served as a template, an Rv2299cD2D3-ESAT6-Rv2882cD2-Rv2005cD3-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c_D2D3_NdeI_F : AAGGAGATATACATATGTCGATGAAGGCGCTG
Rv2299c_D2D3_R : CAAGGTACGCGCGAGACG
ESAT-6_F :
   CGTCTCGCGCGTACCTTGGAATTCATGACAGAGCAGCAGTGG
ESAT-6_R : TGCGAACATCCCAGTGAC
Rv2882c_D2_HindIII _F :
   GATGTTCGCAAAGCTTATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_R1 : GCTAGCCGACTGCCGTGCCACGAT
Rv3463_D2_F1 : GCACGGCAGTCGGCTAGCATGGCACTGGGCCCCCG
Rv3463_NotI_R : TGCTCGAGTGCGGCCGCAGTCAGTCGGAGCGGCTT

### (9) pET-22b(+)_Rv2299cD2D3-ESAT6-Rv2005cD3-Rv1605-Rv3463D2

While Rv2299c D2D3, ESAT6, Rv1605, Rv2005cD3, and Rv3463D2 DNA obtained in the same manner as described above served as a template, an Rv2299cD2D3-ESAT6-Rv2005cD3-Rv1605-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c_D2D3_NdeI_F : AAGGAGATATACATATGTCGATGAAGGCGCTG
Rv2299c_D2D3_R : CAAGGTACGCGCGAGACG
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_D3_R2 : TCTAGACGACTGCCGTGCCACGAT
Rv1605_F : GCACGGCAGTCGTCTAGAATGTATGCCGACCGTGAC
Rv1605_R : GCTAGCTCGCACGGTGATTCCTTC
Rv3463_D2_F2 : ATCACCGTGCGAGCTAGCATGGCACTGGGCCCCCG
Rv3463_NotI_R : TGCTCGAGTGCGGCCGCAGTCAGTCGGAGCGGCTT

### (10) pET-22b(+)_Rv2299cD2D3-ESAT6-Rv3463D2-Rv2882cD2-RpfED1- Rv2145cD2

While Rv2299c D2D3, ESAT6, Rv3463D2, Rv2882cD2, RpfED1, and Rv2145cD2 DNA obtained in the same manner as described above served as a template, an Rv2299cD2D3-ESAT6-Rv3463D2-Rv2882cD2-RpfED1-Rv2145cD2 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2882c_D2_F : CTGACTGCGGCCGCAATGGACGGCGCCCTTATTCG
Rv2882c_D2_XhoI_R :
   GTGGTGGTGGTGCTCGAGGACCTCCAGCAGCTCGCC
RpfE_D1_Rv2882D2_F :
   GAGCTGCTGGAGGTCATGGCCGACGACGCGGGCTTGGAC
RpfE_D1_R : GTTGTAGGCCACGGGCAC
Rv2145c_D2_RpfED1_F : CCCGTGGCCTACAACATGGTCTCGGCGGGGATG
Rv2145c D2 XhoI R :
   GTGGTGGTGGTGCTCGAGGTTTTTGCCCCGGTTGAATTGATC

### (11) pET-22b(+)_Rv2299cD2D3-ESAT6-Rv3463D2-Rv2005cD3

While Rv2299c D2D3, ESAT6, Rv3463D2, and Rv2005cD3 DNA obtained in the same manner as described above served as a template, an Rv2299cD2D3-ESAT6-Rv3463D2-Rv2005cD3 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2005c_D3_F : CTGACTGCGGCCGCAATGTGGAGTGACGTCGAAGT
Rv2005c D3 XhoI R :
   GGTGGTGGTGGTGCTCGAGCGACTGCCGTGCCACGATCAC

### (12) pET-22b(+)_Rv2299cD2D3-ESAT6-Rv3463D2-Rv2005cD3-RpfED1

While Rv2299c D2D3, ESAT6, Rv3463D2, Rv2005cD3, and RpfED1 DNA obtained in the same manner as described above served as a template, an Rv2299cD2D3-ESAT6-Rv3463D2-Rv2005cD3-RpfED1 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2005c_D3_F : CTGACTGCGGCCGCAATGTGGAGTGACGTCGAAGT
Rv2005c_D3_R : CGACTGCCGTGCCACGATCAC
RpfE_D2_Rv2005cD3_F :
   GTGGCACGGCAGTCGATGGCCGACGACGCGGGCTTGGAC
RpfE_D1_XhoI_R : GTGGTGGTGGTGCTCGAGGTTGTAGGCCACGGGCAC

### (13) pET-22b(+)_Rv2299cD2D3-ESAT6-Rv3463D2

While Rv2299cD2D3, ESAT6, and Rv3463D2 DNA obtained in the same manner as described above served as a template, an Rv2299cD2D3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463 D2_NotI_R :
   TGGTGCTCGAGTGCGGCCGCAGTCAGTCGGAGCGGCTTC

### (14) pET-22b(+)_Rv2299cD2D3-ESAT6-Rv3463D2-Rv2882cD2

While Rv2299cD2D3, ESAT6, Rv3463D2, and Rv2882cD2 DNA obtained in the same manner as described above served as a template, an Rv2299cD2D3-ESAT6-Rv3463D2-Rv2882cD2 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2882c_D2_F : CTGACTGCGGCCGCAATGGACGGCGCCCTTATTCG
Rv2882c D2 XhoI R :
   GTGGTGGTGGTGCTCGAGGACCTCCAGCAGCTCGCC

### (15) pET-22b(+)_Rv2299cD2D3-ESAT6-Rv3463D2-Rv2882cD2-Rv2145cD2

While Rv2299cD2D3, ESAT6, Rv3463D2, Rv2882cD2, and Rv2145cD2 DNA obtained in the same manner as described above served as a template, an Rv2299cD2D3-ESAT6-Rv3463D2-Rv2882cD2-Rv2145cD2 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2882c_D2_F : CTGACTGCGGCCGCAATGGACGGCGCCCTTATTCG
Rv2882c_D2_R : GACCTCCAGCAGCTCGCC
Rv2145c_D2_Rv2882cD2_F :
   GAGCTGCTGGAGGTCATGGTCTCGGCGGGGATG
Rv2145c_D2_XhoI_R :
   GTGGTGGTGGTGCTCGAGGTTTTTGCCCCGGTTGAATTGATC

### (16) pET-22b(+)_Rv2299cD2D3-ESAT6-Rv3463D2-Rv2882cD2-RpfED1

While Rv2299cD2D3, ESAT6, Rv3463D2, Rv2882cD2, and RpfED1 DNA obtained in the same manner as described above served as a template, an Rv2299cD2D3-ESAT6-Rv3463D2-Rv2882cD2-RpfED1 DNA fragment with the restriction enzyme sites NdeI and XhoI inserted respectively at the 5' and 3' ends thereof was constructed using an overlapping PCR method, followed by insertion into the pET22b vector. The primers used are as follows.
Rv2299c D2+D3_NdeI_F :
   GAAGGAGATATACATATGAACCTGGTCAAGAAATACTCC
Rv2299c-EcoR-R : GAATTCGGCAAGGTACGCGCGAGACGTTC
ESAT6-EcoR-F : GAATTCGATGACAGAGCAGCAGTGGAAT
ESAT6_R : GGGGCCCAGTGCCATAAGCTTTGCGAACATCCCAGTGACGT
Rv3463 D2_F : ATGGCACTGGGCCCCCGG
Rv3463_D2_R : TGCGGCCGCAGTCAGTCGGAGCG
Rv2882c_D2_F : CTGACTGCGGCCGCAATGGACGGCGCCCTTATTCG
Rv2882c_D2_R : GACCTCCAGCAGCTCGCC
RpfE_D1_Rv2882D2_F :
   GAGCTGCTGGAGGTCATGGCCGACGACGCGGGCTTGGAC
RpfE_D1_XhoI_R : GTGGTGGTGGTGCTCGAGGTTGTAGGCCACGGGCAC

### (17) pET-22b(+)_Rv2882c-Rv2005c-Rv3463

While Rv2882c, Rv2005c, and Rv3463 DNA obtained in the same manner as described above served as a template, an Rv2882c-Rv2005c-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2882c_NdeI_F : AAGGAGATATACATATGATTGATGAGGCTCTCTTCG
Rv2882c_D2_R : GACCTCCAGCAGCTCGC
Rv2005c_F : GCGAGCTGCTGGAGGTCGAATTCATGTCTAAACCCCGCAAG
Rv2005c(HindIII)_R : AAGCTTCGACTGCCGTGCCACGAT
Rv3463 (HindIII)_F : GCACGGCAGTCGAAGCTTATGACCAATTGTGCCGCC
Rv3463_NotI_R : TGCTCGAGTGCGGCCGCAGTCAGTCGGAGCGGCTT

### (18) pET-22b(+)_Rv2882c-Rv2005cD3-ESAT6-Rv3463

While Rv2882c, Rv2005cD3, ESAT6, and Rv3463 DNA obtained in the same manner as described above served as a template, an Rv2882c-Rv2005cD3-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2882c_NdeI_F : AAGGAGATATACATATGATTGATGAGGCTCTCTTCG
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463 (HindIII)_F : GCACGGCAGTCGAAGCTTATGACCAATTGTGCCGCC
Rv3463_NotI_R : GCTCGAGTGCGGCCGCAGTCAGTCGGAGCGGCTT

### (19) pET-22b(+)_Rv2220-Rv2882cD2-Rv2005cD3-ESAT6-Rv3463D2

While Rv2220, Rv2882cD2, Rv2005cD3, ESAT6, and Rv3463D2 DNA obtained in the same manner as described above served as a template, an Rv2220-Rv2882cD2-Rv2005cD3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2220_NdeI_F : AAGGAGATATACATATGACGGAAAAGACGCCC
Rv2220_R : AACGTCGTAGTACAGCGC
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (20) pET-22b(+)_Rv0869c-Rv2882cD2-Rv2005cD3-ESAT6-Rv3463D2

While Rv0869c, Rv2882cD2, Rv2005cD3, ESAT6, Rv3463D2 DNA obtained in the same manner as described above served as a template, an Rv0869c-Rv2882cD2-Rv2005cD3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv0869c_NdeI_F : AAGGAGATATACATATGACACTGACCGCGCTGGG
Rv0869c_NheI_R : CGCCGTCCATGCTAGCGCCACCGATCGCGCTCA
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (21) pET-22b(+)_Rv1605-Rv2882cD2-Rv2005cD3-ESAT6-Rv3463D2

While Rv1605, Rv2882cD2, Rv2005cD3, ESAT6, Rv3463D2 DNA obtained in the same manner as described above served as a template, an Rv1605-Rv2882cD2-Rv2005cD3-ESAT6-Rv3463D2 DNA with the restriction enzyme sites NdeI and NotI inserted respectively at the 5' and 3' ends thereof was constructed using an overlapping PCR method, followed by insertion into the pET22b vector. The primers used are as follows.
Rv1605_NdeI_F : AAGGAGATATACATATGTATGCCGACCGTGACCTTC
Rv1605_NheI_R : CGCCGTCCATGCTAGCTCGCACGGTGATTCCTTC
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (22) pET-22b(+)_Rv2220-ESAT6-Rv3463

While Rv2220, ESAT6, AND Rv3463 DNA obtained in the same manner as described above served as a template, an Rv2220-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2220_NdeI_F : AAGGAGATATACATATGACGGAAAAGACGCCC
Rv2220_EcoRI_R : GCTCTGTCATGAATTCAACGTCGTAGTACAGCGC
ESAT-6_F :
   CGTCTCGCGCGTACCTTGGAATTCATGACAGAGCAGCAGTGG
ESAT-6_R : TGCGAACATCCCAGTGAC
Rv3463_F : GTCACTGGGATGTTCGCAAAGCTTATGACCAATTGTGCCGCC
Rv3463_NotI_R : TGCTCGAGTGCGGCCGCAGTCAGTCGGAGCGGCTT

### (24) pET-22b(+)_Rv0869c-ESAT6-Rv2005cD3-Rv3463

While Rv0869c, ESAT6, Rv2005cD3, and Rv3463 DNA obtained in the same manner as described above served as a template, an Rv0869c-ESAT6-Rv2005cD3-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv0869c_NdeI_F : AAGGAGATATACATATGACACTGACCGCGCTGGG
Rv0869c_EcoRI_R : GCTCTGTCATGAATTCGCCACCGATCGCGCTCAT
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_R : CGACTGCCGTGCCACGAT
Rv3463(NheI)_F : GCACGGCAGTCGGCTAGCATGACCAATTGTGCCGCC
Rv3463_NotI_R : TGCTCGAGTGCGGCCGCAGTCAGTCGGAGCGGCTT

### (25) pET-22b(+)_Rv1605-ESAT6-Rv2005cD3-Rv3463

While Rv1605, ESAT6, Rv2005cD3, and Rv3463 obtained in the same manner as described above served as a template, an Rv1605-ESAT6-Rv2005cD3-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv1605_NdeI_F : AAGGAGATATACATATGTATGCCGACCGTGACCTTC
Rv1605_EcoRI_R : GCTCTGTCATGAATTCTCGCACGGTGATTCCTTC
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_R : CGACTGCCGTGCCACGAT
Rv3463(NheI)_F : GCACGGCAGTCGGCTAGCATGACCAATTGTGCCGCC
Rv3463_NotI_R : TGCTCGAGTGCGGCCGCAGTCAGTCGGAGCGGCTT

### (26) pET-22b(+)_Rv2220-Rv2882cD2-Rv2005cD3-ESAT6-Rv3463D2

While Rv2220, Rv2882cD2, Rv2005cD3, ESAT6, and Rv3463D2 DNA obtained in the same manner as described above served as a template, an Rv2220-Rv2882cD2-Rv2005cD3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2220_NdeI_F : AAGGAGATATACATATGACGGAAAAGACGCCC
Rv2220_R : AACGTCGTAGTACAGCGC
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (27) pET-22b(+)_Rv0869c-Rv2882cD2-Rv2005cD3-ESAT6-Rv3463D2

While Rv0869c, Rv2882cD2, Rv2005cD3, ESAT6, and Rv3463D2 DNA obtained in the same manner as described above served as a template, an Rv0869c-Rv2882cD2-Rv2005cD3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv0869c_NdeI_F : AAGGAGATATACATATGACACTGACCGCGCTGGG
Rv0869c_NheI_R : CGCCGTCCATGCTAGCGCCACCGATCGCGCTCA
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (28) pET-22b(+)_Rv1605-Rv2882cD2-Rv2005cD3-ESAT6-Rv3463D2

While Rv1605, Rv2882cD2, Rv2005cD3, ESAT6, and Rv3463D2 DNA obtained in the same manner as described above served as a template, an Rv1605-Rv2882cD2-Rv2005cD3-ESAT6-Rv3463D2 DNA fragment with the restriction enzyme sites NdeI and NotI inserted respectively at the 5' and 3' ends thereof was constructed using an overlapping PCR method, followed by insertion into the pET22b vector. The primers used are as follows.
Rv1605_NdeI_F : AAGGAGATATACATATGTATGCCGACCGTGACCTTC
Rv1605_NheI_R : CGCCGTCCATGCTAGCTCGCACGGTGATTCCTTC
Rv2882c_D2_F :
   GCGCTGTACTACGACGTTGCTAGCATGGACGGCGCCCTTATTC
Rv2882c_D2_R : GACCTCCAGCAGCTCGC
Rv2005c_D3_F :
   GCGAGCTGCTGGAGGTCTCTAGAATGTGGAGTGACGTCGAAG
Rv2005c_D3_EcoRI_R : GCTCTGTCATGAATTCCGACTGCCGTGCCACGAT
ESAT-6_F :
   CGTCTCGCGCGTACCTTGGAATTCATGACAGAGCAGCAGTGG
ESAT-6_R : TGCGAACATCCCAGTGAC
Rv3463_D2_F3 :
   GTCACTGGGATGTTCGCAAAGCTTATGGCACTGGGCCCCCG
Rv3463_NotI_R : GCTCGAGTGCGGCCGCAGTCAGTCGGAGCGGCTT

### (29) pET-22b(+)_Rv1605-Rv2220-ESAT6-Rv3463

While Rv1605, Rv2220, ESAT6, and Rv3463 DNA obtained in the same manner as described above served as a template, an Rv1605-Rv2220-ESAT6-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv2220_NdeI_F : AAGGAGATATACATATGACGGAAAAGACGCCC
Rv2220_EcoRI_R : GCTCTGTCATGAATTCAACGTCGTAGTACAGCGC
ESAT-6_F :
   CGTCTCGCGCGTACCTTGGAATTCATGACAGAGCAGCAGTGG
ESAT-6_R : TGCGAACATCCCAGTGAC
Rv3463_F : GTCACTGGGATGTTCGCAAAGCTTATGACCAATTGTGCCGCC
Rv3463_NotI_R : TGCTCGAGTGCGGCCGCAGTCAGTCGGAGCGGCTT

### (30) pET-22b(+)_Rv0869c-ESAT6-Rv2005cD3-Rv3463

While Rv0869c, ESAT6, Rv2005cD3, and Rv3463 DNA obtained in the same manner as described above served as a template, an Rv0869c-ESAT6-Rv2005cD3-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv0869c_NdeI_F : AAGGAGATATACATATGACACTGACCGCGCTGGG
Rv0869c_EcoRI_R : GCTCTGTCATGAATTCGCCACCGATCGCGCTCAT
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_R : CGACTGCCGTGCCACGAT
Rv3463(NheI)_F : GCACGGCAGTCGGCTAGCATGACCAATTGTGCCGCC
Rv3463_NotI_R : TGCTCGAGTGCGGCCGCAGTCAGTCGGAGCGGCTT

### (31) pET-22b(+)_Rv1605-ESAT6-Rv2005cD3-Rv3463

While Rv1605, ESAT6, Rv2005cD3, AND Rv3463 DNA obtained in the same manner as described above served as a template, an Rv1605-ESAT6-Rv2005cD3-Rv3463 DNA fragment with the restriction enzyme sites NdeI and NotI inserted respectively at the 5' and 3' ends thereof was constructed using an overlapping PCR method, followed by insertion into the pET22b vector. The primers used are as follows.
Rv1605_NdeI_F : AAGGAGATATACATATGTATGCCGACCGTGACCTTC
Rv1605_EcoRI_R : GCTCTGTCATGAATTCTCGCACGGTGATTCCTTC
ESAT-6_F :
   CGTCTCGCGCGTACCTTGGAATTCATGACAGAGCAGCAGTGG
ESAT-6_R : TGCGAACATCCCAGTGAC
Rv2005c_D3_HindIII_F :
   GATGTTCGCAAAGCTTATGTGGAGTGACGTCGAAG
Rv2005c_R : CGACTGCCGTGCCACGAT
Rv3463(NheI)_F : GCACGGCAGTCGGCTAGCATGACCAATTGTGCCGCC
Rv3463_NotI_R : TGCTCGAGTGCGGCCGCAGTCAGTCGGAGCGGCTT

### 1.3 Production of Recombinant Proteins

All recombinant plasmids prepared as described above were transformed into *Escherichia coli* BL21 cells. The *E*. *coli* cells containing the recombinant plasmids were grown in a shaking incubator at 37°C. When the optical density (OD) at 600 nm reached the desired level, isopropyl-β-D-thiogalactopyranoside (IPTG; ELPIS-Biotech, Daejeon) was added to a final concentration of 1 mM. After 4 to 6 hours, the bacterial cells were harvested by centrifugation and suspended in a solution containing 20 mM Tris-HCl (pH 8.0), 0.5 M NaCl, 5 mM imidazole, 6 M urea, and 1 mM phenylmethylsulfonyl fluoride (Sigma). For the purification of individual proteins, the same composition was used except for the exclusion of urea. After sonication for lysis, the recombinant proteins were purified by nickel-nitrilotriacetic acid (Ni-NTA) agarose chromatography (Qiagen, Chatsworth, CA, USA) according to the manufacturer's instructions. Each purification step was analyzed by sodium dodecyl sulfate-polyacrylamide gel electrophoresis (SDS-PAGE) using Coomassie Brilliant Blue (CB) stain and Western blotting (WB) with an anti-His antibody (Santa Cruz). The purified proteins were concentrated and dialyzed in phosphate-buffered saline (PBS, pH 7.4). PBS was used for dialysis of all individual proteins. To remove endotoxins, the dialyzed proteins were incubated with polymyxin B-agarose (PMB, Sigma) at 4°C for 2 hours. Finally, the purified, endotoxin-free recombinant proteins were filter-sterilized and frozen at -70°C. Protein concentrations were determined using a bicinchoninic acid (BCA) protein assay kit (Pierce, Rockford, IL), with bovine serum albumin (BSA) as the standard. The purity of all proteins was evaluated by Coomassie Blue (CB) staining and Western blot (WB) using an anti-histidine antibody.

### 2. Culture of Mouse Bone Marrow-Derived Dendritic Cells (BMDCs)

Bone marrow-derived dendritic cells (BMDCs) were cultured at 37°C in RPMI 1640 medium (Roswell Park Memorial Institute) supplemented with 10% FBS, 1% antibiotics (Welgene), and 0.1% 2-mercaptoethanol under 5% CO₂. The medium was further supplemented with 5 mM HEPES buffer, 1% MEM solution, 20 ng/ml granulocyte-macrophage colony-stimulating factor (GM-CSF), and 2 ng/ml IL-4. Non-adherent cells and loosely adherent proliferating DC aggregates were harvested on day 7 or 8 and used for further experiments.

### 3. Culture of Mouse Bone Marrow-Derived Macrophages (BMDMs)

BMDMs obtained from the femur and pelvis were cultured in Dulbecco's modified Eagle's medium (DMEM) supplemented with 10% fetal bovine serum (FBS), 50 ng/ml macrophage colony-stimulating factor (M-CSF) (R&D Systems, USA), and 1% antibiotics (Welgene, Korea) in a 5% CO₂, 37°C incubator.

### 4. Preparation of Mtb Strains

Mtb H37Rv (ATCC 27294) and H37Ra (ATCC 25177) were each cultured in 7H9 medium supplemented with 0.5% glycerol, 0.05% Tween-80, 10% oleic acid, albumin, dextrose, and catalase.

### 5. Animal Preparation

Specific pathogen-free female C57BL/6 mice, aged 5-6 weeks, were housed in the biological hazard animal laboratory of the Chungnam National University College of Medicine. The mice were provided with sterilized standard chow under a 12-hour light/12-hour dark cycle. The mice were monitored daily, and no clinical symptoms or diseases were observed during the experiments.

### 6. Cell Infection and Intracellular M. tuberculosis Growth Assay

BMDMs were plated at a density of 1×10⁵ cells per well and cultured. The cells were then treated and infected with Mtb H37Rv (MOI=1) for 4 hours. To remove any extracellular Mtb remaining outside the BMDMs, the cells were treated with the antibiotic amikacin at a concentration of 200 ug/ml for 2 hours, followed by washing with PBS. Specific antigens were then added, and the cells were cultured for 3 or 5 days to measure the intracellular Mtb count.

Alternatively, BMDCs stimulated by each antigen were co-cultured with splenocytes or T-cells isolated from splenocytes at a ratio of 1:10 for 3 days to activate the lymphocytes. These activated lymphocytes were then co-cultured with Mtb-infected BMDMs for 3 or 5 days to measure the intracellular Mtb count.

To measure the intracellular bacterial count, the BMDMs were collected and lysed by treatment with distilled water for 30 minutes. The lysate was then serially diluted and spread onto 7H10 solid medium, which was incubated at 37°C. The number of colonies formed (colony-forming units, CFUs) on the solid medium was analyzed after incubation.

### 7. In vitro T-Cell Proliferation Assay

Responder T-cells involved in the naïve T-cell response were isolated from total mononuclear cells extracted from BALB/c mice using MACS columns (Miltenyi Biotec). OVA-specific CD4+ T-cells as responders were obtained from the splenocytes of OT-2 mice. These T-cells were stained with 1 µM CFSE (Invitrogen). Dendritic cells (2×10⁵ cells/well), treated with OVA peptide in the presence of 10 ug/ml tuberculosis antigen for 24 hours, were co-cultured with CFSE-stained CD4+ T-cells (2×10⁶) at a DC : T cell ratio of 1:10. After 3 or 4 days of co-culture, each batch of T-cells was stained with PerCP-Cy5.5-conjugated anti-CD4 monoclonal antibody and analyzed by flow cytometry. The supernatant was harvested and analyzed for IFN-γ, IL-2, and IL-4 levels by ELISA.

### 8. Enzyme-Linked Immunosorbent Assay (ELISA)

Cytokines produced after stimulating BMDMs or BMDCs with antigens and cytokines produced under various conditions were detected using a sandwich enzyme-linked immunosorbent assay. The cytokines TNF-α, IL-1β, IFN-γ, IL-2, IL-4, and IL-12p70 were measured in the culture media. The analysis of cytokines in the culture media was performed according to the manufacturers' instructions (eBioscience and BD Biosciences). The levels of cytokines released into the culture media were determined by reading the absorbance at 450 nm using a microplate reader. The concentration of cytokines was calculated using a standard curve of recombinant cytokines, and the results were expressed in picograms per milliliter.

### Preparation of BCG-Cell Wall Skeleton (BCG-CWS) and Covalent Binding with Proteins

After autoclaving the cultured BCG strains, BCG-CWS was prepared following the method described by Paik et al. (PMID: 20937311). The final prepared CWS was suspended in 2-propanol (100%) and stored in a freezer. Covalent binding with proteins was performed following the method described by Baik et al. (PLOS One, 2019, PMID: 30849108). CWS was suspended in coupling buffer [20 mM EDC and 50 mM NHS/100% 2-propanol; N-hydroxysulfosuccinimide (NHS), 1-ethyl-3-(3-dimethylaminopropyl) carbodiimide hydrochloride (EDC)] and mixed with a predetermined amount of protein. The mixture was incubated overnight in a refrigerator. Unbound proteins were washed away three times with a suspension buffer (0.02% Tween 80 and 1% ethanol/PBS). The final product was suspended in the suspension buffer, and the protein concentration was measured for use as a vaccine antigen.

### 10. Mixing of Protein with DDA/MPL

The mixture of protein with dimethyl dioctadecylammonium bromide (DDA) and monophosphoryl lipid A (MPL) was prepared following the method of Andersen et al. (PMID: 10639447). Five micrograms of protein, 250 µg of DDA, and 25 µg of MPL were mixed and then added with 0.2% triethylamine to form a final volume of 200 µl. The mixture was then heated in a 70°C water bath for 30 seconds, followed by sonication for 30 seconds. This process was repeated 2 to 3 times. The protein-DDA-MPL mixture was prepared just before use.

### 11. Vaccine Experiments

In the prophylactic vaccine experiments, the test vaccine composition was administered subcutaneously three times. Four or six weeks later, the mice were challenged with Mtb (H37Ra or H37Rv), and after a predetermined period, the bacterial count in the organs was measured.

For the therapeutic vaccine experiments, the mice were first anesthetized with 1.2% 2,2,2-tribromoethanol (Avertin). A small midline incision was made to expose the trachea, and 50 µl of saline containing Mtb was administered intratracheally (IT). After 3 weeks of infection, the mice were treated with "short-term" chemotherapy using isoniazid (INH, 0.1 g/L) and rifampin (RIF, 0.1 g/L), which was provided freely in drinking water for 4 weeks. The vaccine composition was administered three times at regular intervals following the start of treatment, and the bacterial count in the lungs was measured after a predetermined time. The bacterial count in the lungs was determined by euthanizing the mice with CO₂, excising and homogenizing the lungs, and plating the lung homogenate in serial dilutions on Middlebrook 7H10 agar (Difco Laboratories, Detroit, MI) supplemented with 10% OADC (Difco Laboratories), amphotericin B (Sigma-Aldrich, St. Louis), and 2 µg/ml 2-thiophenecarboxylic acid hydrazide (Sigma-Aldrich). The plates were incubated at 37°C for 4 weeks, and the colonies were counted. Data for CFUs and lung inflammation were expressed as log₁₀ CFU ± interquartile range (IQR).

### 12. Statistical Analysis

All experiments were repeated at least three times. Statistical significance for comparisons between samples was determined using Tukey's multiple comparison test in the statistical software (GraphPad Prism Software, version 4.03; GraphPad Software, San Diego, CA). Data in the graphs were presented as the mean ± SEM, and differences from each value were considered statistically significant at * p <0.05, ** p <0.01 **p** < 0.01, or *** p <0.001.

### <EXAMPLE 1: Identification of Active Sites through Immunoassay of Mtb Antigen Domains>

To construct a highly effective tuberculosis vaccine, it is advantageous to fuse multiple proteins of strong immunological activity. However, when multiple proteins are fused, the molecular weight increases, making purification difficult, and the proteins are more prone to denaturation during the purification process. Even if a large molecular weight vaccine is effective, it faces significant challenges in standardizing purification methods during commercialization, leading to prolonged development times and a high likelihood of failure due to difficulties in producing recombinant proteins. Therefore, there is a need for the development of vaccines that maintain high efficacy while having a smaller molecular weight to improve production efficiency.

The inventors previously reported that a fusion protein composed of the dendritic cell-activating protein Rv2299c (molecular weight 72 kDa) and the T-cell-activating protein ESAT6 (molecular weight 10 kDa) showed very high efficacy as a BCG booster tuberculosis vaccine (Ref, Oncotarget. PMID: 28193909). However, the Rv2299c-ESAT6 fusion protein has a molecular weight of approximately 80 kDa, which is relatively large. Therefore, the inventors investigated the possibility of reducing the molecular weight while maintaining the same protective effect and adding new immune-active proteins to the fusion. This led to the present study, which aimed to identify the highly immune-active regions from the proteins developed by the inventors and create a vaccine composed of these active sites.

### 1.1. Identification of Active Sites through Immunoassay of Rv2299c Protein by Domain (Region)

First, in order to identify an active domain in the dendritic cell-activating protein Rv2299c, the protein was divided into three regions, and each recombinant protein was expressed and purified from *E*. *coli,* as shown in FIG. 1A. When each domain was applied to bone marrow-derived dendritic cells (BMDCs) from C57BL/6 mice, the D2 and D3 regions induced the production of IL-1β and IL-12, whereas the D1 region did not induce the production of either IL-1β or IL-12, as shown in FIG. 1B. Additionally, as shown in FIG. 1C, BMDCs activated by each protein were co-cultured with naïve T-cells isolated from the spleens of C57BL/6 mice for 3 days and then added to Mtb-infected bone marrow-derived macrophages (BMDMs). After 3 or 5 days, the intracellular growth of Mtb in BMDMs was measured by determining the colony-forming units (CFUs) and is represented in FIG. 1D. As shown in FIG. 1D, T-cells activated by BMDCs stimulated with full-length protein, D2, or D3 regions of Rv2299c significantly inhibited intracellular bacterial growth, whereas the D1 region did not inhibit intracellular bacterial growth. Furthermore, as shown in FIG. 1E, the production of IFN-γ and IL-17 was proportional to the inhibition of bacterial growth.

Based on these results, the anti-tuberculosis activity of the D2 and D3 regions of the Rv2299c protein, which showed strong bacterial growth inhibition, was re-evaluated and is shown in FIG. 2. As shown in FIG. 2, T-cells activated by BMDCs stimulated with the D3 region showed significantly higher intracellular bacterial growth inhibition compared to the D2 region, along with higher IFN-γ production. On the other hand, the D2 region specifically increased IL-2 production significantly.

On the basis of these results, a recombinant protein combining the D2 and D3 regions of Rv2299c was created, as shown in FIG. 3A. T-cells activated by BMDCs stimulated with each protein were added to Mtb-infected BMDMs and cultured for 3 days. As shown in FIG. 3B, the full-length Rv2299c protein, the D3 region, and the D2+D3 region exhibited strong anti-tuberculosis activity. These results suggest that using only the D2+D3 or D3 domains, excluding the D1 region, can induce the same vaccine efficacy as the full-length Rv2299c protein.

The amino acid sequences of full-length Rv2299c, and the D1, D2, D3, D2+D3, and ESAT6 regions are given in Table 2.

**[TABLE 2]**

| **Domain (No. of AA)** | **Amino acid sequence** | **SEQ ID NO:** |
|---|---|---|
| Rv2299c (647) | MNAHVEQLEFQAEARQLLDLMVHSVYSNKDAFLRELISNAS DALDKLRIEALRNKDLEVDTSDLHIEIDADKAARTLTVRDN GIGMAREEVVDLIGTLAKSGTAELRAQLREAKNAAASEELI GQFGIGFYSSFMVADKVQLLTRKAGESAATRWESSGEGTYT IESVEDAPQGTSVTLHLKPEDAEDDLHDYTSEWKIRNLVKK YSDFIAWPIRMDVERRTPASQEEGGEGGEETVTIETETLNS MKALWARPKEEVSEQEYKEFYKHVAHAWDDPLEIIAMKAEG TFEYQALLFIPSHAPFDLFDRDAHVGIQLYVKRVFIMGDCD QLMPEYLRFVKGVVDAQDMSLNVSREILQQDRQIKAIRRRL | 1 |
| | TKKVLSTIKDVQSSRPEDYRTFWTQFGRVLKEGLLSDIDNR ETLLGISSFVSTYSEEEPTTLAEYVERMKDGQQQIFYATGE TRQQLLKSPHLEAFKAKGYEVLLLTDPVDEVWVGMVPEFDG KPLQSVAKGEVDLSSEEDTSEAEREERQKEFADLLTWLQET LSDHVKEVRLSTRLTESPACLITDAFGMTPALARIYRASGQ EVPVGKRILELNPSHPLVTGLRQAHQDRADDAEKSLAETAE LLYGTALLAEGGALEDPARFAELLAERLARTL | |
| Rv2299cD1 (245) | MNAHVEQLEFQAEARQLLDLMVHSVYSNKDAFLRELISNAS DALDKLRIEALRNKDLEVDTSDLHIEIDADKAARTLTVRDN GIGMAREEVVDLIGTLAKSGTAELRAQLREAKNAAASEELI GQFGIGFYSSFMVADKVQLLTRKAGESAATRWESSGEGTYT IESVEDAPQGTSVTLHLKPEDAEDDLHDYTSEWKIRNLVKK YSDFIAWPIRMDVERRTPASQEEGGEGGEETVTIETETLN | 2 |
| Rv2299cD2 (169) | SMKALWARPKEEVSEQEYKEFYKHVAHAWDDPLEIIAMKAE GTFEYQALLFIPSHAPFDLFDRDAHVGIQLYVKRVFIMGDC DQLMPEYLRFVKGVVDAQDMSLNVSREILQQDRQIKAIRRR LTKKVLSTIKDVQSSRPEDYRTFWTQFGRVLKEGLLSDIDN RETLL | 3 |
| Rv2299cD3 (234) | LGISSFVSTYSEEEPTTLAEYVERMKDGQQQIFYATGETRQ QLLKSPHLEAFKAKGYEVLLLTDPVDEVWVGMVPEFDGKPL QSVAKGEVDLSSEEDTSEAEREERQKEFADLLTWLQETLSD HVKEVRLSTRLTESPACLITDAFGMTPALARIYRASGQEVP VGKRILELNPSHPLVTGLRQAHQDRADDAEKSLAETAELLY GTALLAEGGALEDPARFAELLAERLARTL | 4 |
| Rv2299cD2D 3 (402) | SMKALWARPKEEVSEQEYKEFYKHVAHAWDDPLEIIAMKAE GTFEYQALLFIPSHAPFDLFDRDAHVGIQLYVKRVFIMGDC DQLMPEYLRFVKGVVDAQDMSLNVSREILQQDRQIKAIRRR LTKKVLSTIKDVQSSRPEDYRTFWTQFGRVLKEGLLSDIDN RETLLGISSFVSTYSEEEPTTLAEYVERMKDGQQQIFYATG ETRQQLLKSPHLEAFKAKGYEVLLLTDPVDEVWVGMVPEFD GKPLQSVAKGEVDLSSEEDTSEAEREERQKEFADLLTWLQE TLSDHVKEVRLSTRLTESPACLITDAFGMTPALARIYRASG QEVPVGKRILELNPSHPLVTGLRQAHQDRADDAEKSLAETA ELLYGTALLAEGGALEDPARFAELLAERLARTL | 5 |
| ESAT6 | MTEQQWNFAGIEAAASAIQGNVTSIHSLLDEGKQSLTKLAA | 6 |
| (95) | AWGGSGSEAYQGVQQKWDATATELNNALQNLARTISEAGQA MASTEGNVTGMFA | |

### 1.2. Identification of Immune-Active Domains of RpfE Protein

The dendritic cell-activating RpfE protein was divided into D1 and D2 regions, as shown in FIG. 4A, and recombinant proteins for each domain were produced in *E*. *coli* and purified, as shown in FIG. 4B. The cytokines produced by BMDCs stimulated with each domain were measured and the results are depicted in FIG. 4C. The results indicated that stimulation with the D1 and D2 domains of the RpfE protein induced similar levels of IL-1β and IL-12, but IL-23 levels were significantly lower when BMDCs were stimulated with the D2 domain. Next, the anti-tuberculosis activity of T-cells stimulated by BMDCs activated with the purified domains was measured, and the results are depicted in FIG. 4D. As shown in FIG. 4D, the full-length RpfE protein and the D1 domain of RpfE exhibited similar anti-tuberculosis activity. The concentrations of the cytokines IFN-γ, IL-2, and IL-17 produced during this process were measured and are shown in FIG. 4E. As illustrated in FIG. 4E, all of the RpfE, D1, and D2 domains showed similar effects, but IL-17 production was significantly lower for the D2 domain. Furthermore, after BMDCs matured by each domain were co-cultured with T-cells isolated from the spleens of mice infected with BCG or Mtb H37Ra strain, the cytokines produced were measured. As shown in FIG. 5, there was no significant difference in IFN-γ production, but IL-2 and IL-17 production was significantly lower when treated with the D2 domain. These results suggest that the D1 domain of RpfE alone is sufficient to induce adequate vaccine efficacy.

The amino acid sequences of the full-length RpfE protein and the D1 and D2 domains are listed in Table 3, below.

**[TABLE 3]**

| **Domain (No. of AA)** | **Amino acid sequence** | **SEQ ID NO:** |
|---|---|---|
| RpfE (172) | MKNARTTLIAAAIAGTLVTTSPAGIANADDAGLDPNA AAGPDAVGFDPNLPPAPDAAPVDTPPAPEDAGFDPNL PPPLAPDFLSPPAEEAPPVPVAYSVNWDAIAQCESGG NWSINTGNGYYGGLRFTAGTWRANGGSGSAANASREE QIRVAENVLRSQGIRAWPVCGRRG | 7 |
| RpfED1 (71) | ADDAGLDPNAAAGPDAVGFDPNLPPAPDAAPVDTPPA PEDAGFDPNLPPPLAPDFLSPPAEEAPPVPVAYN | 8 |
| RpfED2 (74) | VNWDAIAQCESGGNWS INTGNGYYGGLRFTAGTWRAN GGSGSAANASREEQIRVAENVLRSQGIRAWPVCGRRG | 9 |

### 1.3. Identification of Immune-Active Domains of Rv2882c Protein

The macrophage-activating Rv2882c protein was divided into the D1 and D2 regions, as shown in FIG. 6A, and cloned into expression vectors, after which the recombinant proteins were produced in *E. coli.* However, during this process, the D2 region was not expressed (FIG. 6A). When Mtb-infected BMDMs were stimulated with the full-length Rv2882c protein or the D1 region, there was no significant inhibition of intracellular bacterial growth, as shown in FIG. 6B. However, when mouse splenocytes were added, the bacterial growth in BMDMs stimulated with the full-length Rv2882c protein was significantly inhibited, but no difference in bacterial growth was observed when BMDMs were stimulated with the D1 region and splenocytes were added. This suggests that the D2 domain of the Rv2882c protein is the region with anti-tuberculosis efficacy.

The amino acid sequences of the full-length Rv2882c protein and the D1 and D2 regions are listed in Table 4, below.

**[TABLE 4]**

| **Domain (No. of AA)** | **Amino acid sequence** | **SEQ ID NO:** |
|---|---|---|
| Rv2882c (185) | MIDEALFDAEEKMEKAVAVARDDLSTIRTGRANPGMFS RITIDYYGAATPITQLASINVPEARLVVIKPYEANQLR AIETAIRNSDLGVNPTNDGALIRVAVPQLTEERRRELV KQAKHKGEEAKVSVRNIRRKAMEELHRIRKEGEAGEDE VGRAEKDLDKTTHQYVTQIDELVKHKEGELLEV | 10 |
| Rv2882cD1 (93) | MIDEALFDAEEKMEKAVAVARDDLSTIRTGRANPGMFS RITIDYYGAATPITQLASINVPEARLVVIKPYEANQLR AIETAIRNSDLGVNPTN | 11 |
| Rv2882cD2 (92) | DGALIRVAVPQLTEERRRELVKQAKHKGEEAKVSVRNI RRKAMEELHRIRKEGEAGEDEVGRAEKDLDKTTHQYVT QIDELVKHKEGELLEV | 12 |

### 1.4. Identification of Immune-Active Domains of Rv2145c Protein

The present inventors have previously reported that the macrophage-activating protein Rv2145c induces the production of IL-10, which promotes the growth of *M. tuberculosis,* rather than exhibiting anti-tuberculosis activity (Ref, Front Microbiol, PMID: 34017340). Additionally, an analysis of the anti-tuberculosis activity of Rv2145c revealed that the C-terminal region, specifically amino acids 91 to 260 (D2 region), is the domain that exhibits activity. In the development of tuberculosis vaccines, the inventors selected the Rv2145c protein to analyze vaccine efficacy by adding pathogenicity-related domains in addition to immune-active regions.

The amino acid sequences of the full-length Rv2145c protein and the D1 and D2 regions are listed in Table 5, below.

**[TABLE 5]**

| **Domain (No. of AA)** | **Amino acid sequence** | **SEQ ID NO:** |
|---|---|---|
| Rv2145c (260) | MPLTPADVHNVAFSKPPIGKRGYNEDEVDAFLDLVENE LTRLIEENSDLRQRINELDQELAAGGGAGVTPQATQAI PAYEPEPGKPAPAAVSAGMNEEQALKAARVLSLAQDTA DRLTNTAKAESDKMLADARANAEQILGEARHTADATVA EARQRADAMLADAQSRSEAQLRQAQEKADALQADAERK HSEIMGTINQQRAVLEGRLEQLRTFEREYRTRLKTYLE SQLEELGQRGSAAPVDSNADAGGFDQFNRGKN | 13 |
| Rv2145cD1 (90) | MPLTPADVHNVAFSKPPIGKRGYNEDEVDAFLDLVENE LTRLIEENSDLRQRINELDQELAAGGGAGVTPQATQAI PAYEPEPGKPAPAA | 14 |
| Rv2145cD2 (170) | VSAGMNEEQALKAARVLSLAQDTADRLTNTAKAESDKM LADARANAEQILGEARHTADATVAEARQRADAMLADAQ SRSEAQLRQAQEKADALQADAERKHSEIMGTINQQRAV LEGRLEQLRTFEREYRTRLKTYLESQLEELGQRGSAAP VDSNADAGGFDQFNRGKN | 15 |

### 1.5. Identification of Immune-Active Domains of Rv2005c Protein

The Rv2005c protein is related to the reactivation of *Mycobacterium tuberculosis* and is known to activate BMDCs (Ref. Vaccine 2020, PMID: 32664238). To identify the active regions of Rv2005c, the protein was divided into three domains, as shown in FIG. 7A, and recombinant proteins were produced. However, the D2 region could not be expressed and was therefore not produced. When BMDMs infected with Mtb were stimulated with the full-length Rv2005c protein, the D1 region, or the D3 region, or added with mouse splenocytes, the intracellular bacterial inhibition effect was measured after 72 hours. The D3 domain showed the highest anti-tuberculosis activity (FIG. 7B).

The amino acid sequences of the full-length Rv2005c protein and the D1 and D2 regions are listed in Table 6.

**[TABLE 6]**

| **Domain (No. of AA)** | **Amino acid sequence** | **SEQ ID NO:** |
|---|---|---|
| Rv2005c (295) | MSKPRKQHGVVVGVDGSLESDAAACWGATDAAMRNIPL TVVHVVNADVATWPPMPYPETWGVWQEDEGRQIVANAV KLAKEAVGADRKLSVKSELVFSTPVPTMVEISNEAEMV VLGSSGRGALARGLLGSVSSSLVRRAGCPVAVIHSDDA VIPDPQHAPVLVGIDGSPVSELATAVAFDEASRRGVEL IAVHAWSDVEVVELPGLDFSAVQQEAELSLAERLAGWQ ERYPDVPVSRVVVCDRPARKLVQKSASAQLVVVGSHGR GGLTGMLLGSVSNAVLHAARVPVIVARQS | 16 |
| Rv2005cD1 (110) | MSKPRKQHGVVVGVDGSLESDAAACWGATDAAMRNIPL TVVHVVNADVATWPPMPYPETWGVWQEDEGRQIVANAV KLAKEAVGADRKLSVKSELVFSTPVPTMVEISNE | 17 |
| Rv2005cD3 (100) | WSDVEVVELPGLDFSAVQQEAELSLAERLAGWQERYPD VPVSRVVVCDRPARKLVQKSASAQLVVVGSHGRGGLTG MLLGSVSNAVLHAARVPVIVARQS | 18 |

### 1.6. Identification of Immune-Active Domains of Rv3463 Protein

The macrophage-activating Rv3463 protein was also divided into D1 and D2 regions, cloned into expression vectors, and recombinant proteins were produced in *E*. *coli.* However, the D1 region was not expressed (FIG. 8A). BMDMs infected with Mtb were stimulated with the full-length Rv3463 protein and the D2 region, and intracellular bacterial growth was measured after 72 hours. Both the full-length Rv3463 protein and the D2 region inhibited intracellular bacterial growth to the same extent (FIG. 8B). In this case, bacterial growth was inhibited solely by protein treatment without the addition of T cells. These results suggest that the D2 domain of Rv3463 alone is sufficient to exhibit anti-tuberculosis activity.

The amino acid sequences of the full-length Rv3463 protein and the D1 and D2 regions are listed in Table 7.

**[TABLE 7]**

| **Domain (No. of AA)** | **Amino acid sequence** | **SEQ ID NO:** |
|---|---|---|
| Rv3463 (285) | MTNCAAGKPSSGPNLGRFGSFGRGVTPQQATEIEALGY GAVWVGGSPPAALSWVEPILQATTTLCVATGIVNIWSA PAQRVAESFHRIEAAYPGRFLLGIGVGHAEMISEYRKP YNALVEYLDRLDDYGVPANRRVVAALGPRVLGLSARRS AGAHPYLTTPEHTARARELIGPSAFLAPEHKVVLTTDS ARARTVGRQALDMYFNLANYRNNWKRLGFTDDEVSRPG SDRLVDAVVAYGTPDAIAARLNEHLLAGADHVPIQVLT EDDNLVSALTELAKPLRLT | 19 |
| Rv3463D1 (138) | MTNCAAGKPSSGPNLGRFGSFGRGVTPQQATEIEALGY GAVWVGGSPPAALSWVEPILQATTTLCVATGIVNIWSA PAQRVAESFHRIEAAYPGRFLLGIGVGHAEMISEYRKP YNALVEYLDRLDDYGVPANRRVVA | 20 |
| Rv3463D2 (147) | ALGPRVLGLSARRSAGAHPYLTTPEHTARARELIGPSA FLAPEHKVVLTTDSARARTVGRQALDMYFNLANYRNNW KRLGFTDDEVSRPGSDRLVDAVVAYGTPDAIAARLNEH LLAGADHVPIQVLTEDDNLVSALTELAKPLRLT | 21 |

In addition, amino acid sequences of Mtb-derived antigens used in the present disclosure are listed in Table 8, below.

**[TABLE 8]**

| **Domain (No. of AA)** | **Amino acid sequence** | **SEQ ID NO:** |
|---|---|---|
| Rv1605 (267) | MYADRDLPGAGGLAVRVIPCLDVDDGRVVKGVNFENLR DAGDPVELAAVYDAEGADELTFLDVTASSSGRATMLEV VRRTAEQVFIPLTVGGGVRTVADVDSLLRAGADKVAVN TAAIACPDLLADMARQFGSQCIVLSVDARTVPVGSAPT PSGWEVTTHGGRRGTGMDAVQWAARGADLGVGEILLNS MDADGTKAGFDLALLRAVRAAVTVPVIASGGAGAVEHF APAVAAGADAVLAASVFHFRELTIGQVKAALAAEGITV | 22 |
| | R | |
| Rv2220 (478) | MTEKTPDDVFKLAKDEKVEYVDVRFCDLPGIMQHFTIP ASAFDKSVFDDGLAFDGSSIRGFQSIHESDMLLLPDPE TARIDPFRAAKTLNINFFVHDPFTLEPYSRDPRNIARK AENYLISTGIADTAYFGAEAEFYIFDSVSFDSRANGSF YEVDAISGWWNTGAATEADGSPNRGYKVRHKGGYFPVA PNDQYVDLRDKMLTNLINSGFILEKGHHEVGSGGQAEI NYQFNSLLHAADDMQLYKYIIKNTAWQNGKTVTFMPKP LFGDNGSGMHCHQSLWKDGAPLMYDETGYAGLSDTARH YIGGLLHHAPSLLAFTNPTVNSYKRLVPGYEAPINLVY SQRNRSACVRIPITGSNPKAKRLEFRSPDSSGNPYLAF SAMLMAGLDGIKNKIEPQAPVDKDLYELPPEEAASIPQ TPTQLSDVIDRLEADHEYLTEGGVFTNDLIETWISFKR ENEIEPVNIRPHPYEFALYYDV | 23 |
| Rv0869c (360) | MTLTALGMPALRSRTNGIADPRVVPTTGPLVDTFGRVA NDLRVSLTDRCNLRCSYCMPERGLRWLPGEQLLRPDEL ARLIHIAVTRLGVTSVRFTGGEPLLAHHLDEVVAATAR LRPRPEISLTTNGVGLARRAGALAEAGLDRVNVSLDSI DRAHFAAITRRDRLAHVLAGLAAAKAAGLTPVKVNAVL DPTTGREDVVDLLRFCLERGYQLRVIEQMPLDAGHSWR RNIALSADDVLAALRPHFRLRPDPAPRGSAPAELWLVD AGPNTPRGRFGVIASVSHAFCSTCDRTRLTADGQIRSC LFSTEETDLRRLLRGGADDDAIEAAWRAAMWSKPAGHG INAPDFIQPDRPMSAIGG | 24 |

### <EXAMPLE 2: In Vitro Immunological Activity of Fusion Proteins of Rv2299c Domains, ESAT6, and RpfE Active Domains >

Based on the results of Example 1, fusion proteins composed of various domains of Rv2299c and ESAT6 were investigated for immunological activity.

As shown in FIG. 9, fusion proteins were produced by cloning the genes into expression vectors and transforming the corresponding plasmids into *E. coli.* These included Rv2299cD2D3-ESAT6, Rv2299cD3-ESAT6, and Rv2299cD2D3-ESAT6-RpfED1, which were fusions of the D2 and D3 domains of Rv2299c (excluding D1) with ESAT6, and a fusion of the RpfE1 D1 active region with Rv2299cD2D3-ESAT6. The recombinant proteins were then purified.

As used herein, the term "Rv2299cD2D3-ESAT6 fusion protein" refers to a polypeptide in which the amino acid sequences of SEQ ID NOS: 5 and 6 are sequentially fused to each other, with an end of Rv2299cD2D3 linked to an end of ESAT6. The Rv2299cD2D3-ESAT6 fusion protein was produced according to the recombinant protein production method described in Example 1.2 using pET-22b(+)_Rv2299cD2D3-ESAT6.

As used herein, the term "fusion protein" containing two or more antigens or immune-active regions derived from *Mycobacterium tuberculosis* refers to a polypeptide in which amino acid sequences of corresponding antigens derived from Mtb or immune-active regions thereof are sequentially linked and fused together.

Since the recombinant proteins were purified from *E. coli,* examination was made to see whether the proteins were contaminated with LPS, which is a constituent of E. coli cell mass. As shown in FIG. 10, the absence of LPS contamination was confirmed and even at LPS concentrations of 100 ng/ml, there was no significant difference in TNF-α and IL-1β levels compared to the controls. Additionally, the levels of TNF-α and IL-1β increased upon treatment with Rv2299cD3-ESAT6 (D3-E6), Rv2299cD2D3-ESAT6 (D2D3-E6), and Rv2299cD2D3-ESAT6-RpfED1 (D2D3-E6-RpfED1), demonstrating the absence of LPS contamination.

The anti-tuberculosis activity of the three fusion proteins Rv2299c-ESAT6, Rv2299cD2D3-ESAT6, and Rv2299cD3-ESAT6 was evaluated, and the results are depicted in FIG. 11. T-cells stimulated by BMDCs matured with each protein were co-cultured with Mtb-infected BMDMs for 72 hours. The Mtb cells were counted and are depicted in FIG. 11A. As shown in FIG. 11A, all three fusion proteins exhibited similar anti-tuberculosis activity. Additionally, IL-2, IL-17, and IFN-γ production was measured and depicted in FIG. 11B. As shown in FIG. 11B. IL-2 and IL-17 levels were similarly increased by all three fusion proteins, while IFN-γ production was highest in the Rv2299c-ESAT6 group. Although Rv2299cD2D3-ESAT6 and Rv2299cD3-ESAT6 produced lower levels of IFN-γ compared to Rv2299c-ESAT6, they still effectively increased IFN-γ production.

Then, the fusion protein in which the active D1 domain of RpfE, known to be associated with Mtb reactivation, was fused with Rv2299cD2D3-ESAT6 was measured for anti-tuberculosis activity. BMDCs were stimulated with the three fusion proteins Rv2299c-ESAT6, Rv2299cD2D3-ESAT6, and Rv2299cD2D3-ESAT6-RpfED1 for 24 hours, and measured for production of IL-1β, TNF-α, IL-12, and IL-23 by ELISA. The measurements are depicted in FIG. 12. As shown in FIG. 12, all the three fusion proteins significantly induced the production of IL-1β, TNF-α, and IL-12, while IL-23 production was most strongly induced by Rv2299cD2D3-ESAT6-RpfED1.

The T-cell activation response of BMDCs matured with each fusion protein was measured using ovalbumin (OVA) peptide-specific transgenic T-cells, as shown in FIG. 13A. FIG. 13B shows the results of fluorescence-activated cell sorting (FACS) after BMDCs were treated with each of the fusion proteins Rv2299c-ESAT6, Rv2299D3-ESAT6, Rv2299cD2D3-ESAT6 and Rv2299cD2D3-ESAT6-RpfED1 and OVA peptide for 24 hours and then co-cultured with BMDCs activated by pretreatment of OVA peptide-specific T cells with 5,6-carboxyfluorescein diacetate succinimidyl ester (CFSE). As shown in FIG. 13B, BMDCs matured with each of the four fusion proteins Rv2299c-ESAT6, Rv2299cD3-ESAT6, Rv2299cD2D3-ESAT6, and Rv2299cD2D3-ESAT6-RpfED1 all induced T-cell proliferation and Th1 responses. The cytokines produced during this process were measured and are depicted in FIG. 13C. FIG. 13C shows that all four fusion proteins promoted the production of IFN-γ, IL-17, IL-2, and TNF-α, with Rv2299cD2D3-ESAT6-RpfED1 particularly increasing the production of IFN-γ, IL-17, and TNF-α.

Examination was made to see the intracellular bacterial inhibition effect of Rv2299c-ESAT6, Rv2299D3-ESAT6, Rv2299cD2D3-ESAT6, and Rv2299cD2D3-ESAT6-RpfED1. In this regard, T-cells activated by BMDCs matured with the four fusion proteins were added to Mtb-infected BMDMs. As shown in FIG. 14A, all the four fusion proteins demonstrated an inhibitory effect on bacterial growth, with Rv2299cD2D3-ESAT6 and Rv2299cD2D3-ESAT6-RpfED1 showing the most significant inhibitory effects. Notably, Rv2299cD2D3-ESAT6-RpfED1 nearly eliminated Mtb from infected BMDMs. The cytokine concentrations produced during this process are shown in FIG. 14B. As shown in FIG. 14B, all the four fusion proteins increased the production of IFN-γ, IL-17, and IL-2, with Rv2299cD2D3-ESAT6-RpfED1 producing the highest levels, consistent with the bacterial elimination results.

### <EXAMPLE 3: Evaluation of Vaccine Efficacy of Antigens Conjugated with Rv2299c Domain, ESAT6, RpfE, and BCG Cell Wall Skeleton (BCG-CWS)>

The BCG cell wall skeleton (BCG-CWS) has been reported as a delivery vehicle capable of inducing a Th1 response in experiments using OVA (Ref. Vaccine, 2010, PMID: 20937311). In previous research, the present inventors reported that when the tuberculosis protein Ag85B was conjugated to BCG-CWS and used to immunize mice, a challenge with Mtb showed similar protective effects to BCG at 6 weeks, with continued protection lasting up to 32 weeks (3.2 log bacteria were detected in the lungs and spleen). However, the protective effect of BCG alone was lost by 32 weeks (Ref. PLOS One, 2019, PMID 30849108). Based on these findings, the efficacy of tuberculosis vaccines in which the fusion proteins from the previous examples were conjugated with BCG-CWS were evaluated.

### 3.1. Vaccine Efficacy of Rv2299c-ESAT6 and BCG-CWS Conjugated Antigens

The fusion vaccines of immune-active proteins or domains described above were covalently bonded to BCG-CWS and then were evaluated for tuberculosis vaccine efficacy. Rv2299c-ESAT6, which has been reported to have a BCG booster effect (Ref. Oncotarget 2017, PMID: 28193909), was used. FIG. 15A shows the schematic of the vaccine efficacy evaluation experiment. C57BL/6 mice were divided into seven groups (G1: infection control, G2: antigen control BCG-CWS (10µg), G3: antigen control BCG-CWS/dimethyldioctadecylammonium (DDA) (10µg/250µg), G4: Rv2299c-ESAT6-BCG-CWS (7.5µg/10µg), G5: Rv2299c-ESAT6-BCG-CWS/DDA (7.5µg/10µg/250µg), G6: adjuvant control DDA/MPL (250µg/25µg), G7: Rv2299c-ESAT6/DDA/MPL (5µg/250µg/25µg)). Groups G2-G7 were immunized subcutaneously three times, 8, 6, and 4 weeks before Mtb challenge. Four weeks after the final immunization, a non-virulent strain, *M. tuberculosis* H37Ra (1×10⁶ CFU), was administered intratracheally to all groups. Six weeks and 18 weeks after infection, the mice were sacrificed, and the bacterial count in the lungs was measured. The measurements are depicted in FIG. 15B. As shown in FIG. 15B, the combination of BCG-CWS with DDA showed greater protection at 18 weeks than at 6 weeks. The Rv2299c-ESAT6 antigen mixed with DDA/MPL (G5), which is usually used for immunizing animals with a protein antigen subunit vaccine, showed no vaccine efficacy. Most notably, Rv2299c-ESAT6-CWS (G4) demonstrated the highest vaccine efficacy at 6 weeks and induced complete bacterial clearance in the lungs by 18 weeks. These results demonstrate that antigens covalently bonded with BCG-CWS provides superior protective efficacy compared to using adjuvants such as DDA/MPL.

### 3.2. Vaccine Efficacy of BCG-CWS-Conjugated Fusion Proteins of Rv2299c and RpfE Segmented Domains and ESAT6

Based on the results of FIG. 15, the vaccine efficacy of BCG-CWS-conjugated fusion proteins of Rv2299c and RpfE segmented domains and ESAT6 was evaluated in the context of infection with the virulent strain *M. tuberculosis* H37Rv, as shown in FIG. 16A. C57BL/6 mice were divided into six groups (G1: infection control, G2: antigen control BCG (1×10⁵ CFU), G3: antigen control BCG-CWS (10µg), G4: Rv2299c-ESAT6-BCG-CWS (5µg/10µg), G5: Rv2299cD2D3-ESAT6-BCG-CWS (5µg/10µg), G6: Rv2299cD2D3-ESAT6-RpfED1-BCG-CWS (5µg/10µg)). Groups G2-G6 were immunized subcutaneously three times, 10, 8, and 6 weeks before Mtb challenge. Six weeks after the final immunization, all groups were infected with the pathogenic strain *M. tuberculosis* H37Rv (1×10³ CFU) intratracheally. Six weeks and 28 weeks after infection, the mice were sacrificed, and the bacterial count in the lungs was measured, and the measurements are depicted in FIG. 16B. At 6 weeks post-infection, the Rv2299c-ESAT6-CWS (G4), Rv2299cD2D3-ESAT6-CWS (G5), and Rv2299cD2D3-ESAT6-RpfED1-CWS (G6) groups all showed significant bacterial inhibition in the lungs. The Rv2299cD2D3-ESAT6-RpfED1-CWS group showed the greatest bacterial inhibition, with five out of six mice showing no detectable bacteria. By 28 weeks, the mice immunized with Rv2299cD2D3-ESAT6-CWS (G5) and Rv2299cD2D3-ESAT6-RpfED1-CWS (G6) showed no bacterial growth in the lungs. Additionally, gross pathology revealed significantly fewer granulomas in these groups (FIG. 16C). Removing the D1 domain from Rv2299c improved the protective effect. Although the live BCG (G2) showed some efficacy at 6 weeks, its protective effect was lost by 28 weeks. It has been reported that the protective effect of BCG is generally drastically decreased from 10 weeks post vaccination. In contrast, Rv2299c-ESAT6-CWS (G5), Rv2299cD2D3-ESAT6-CWS (G5), and Rv2299cD2D3-ESAT6-RpfED1-CWS (G6) induced near-complete bacterial clearance, demonstrating superior vaccine efficacy compared to both subunit and live vaccines reported to date.

Six weeks after Mtb infection, the mice were sacrificed, and lung and spleen cells were isolated and stimulated with purified protein derivatives (PPD), ESAT6, Rv2299c-ESAT6, Rv2299cD2D3-ESAT6, and Rv2299cD2D3-ESAT6-RpfED1 antigens. The cytokines produced in the culture supernatant were measured and are depicted in FIGS. 17 and 18. As shown in FIGS. 17 and 18, the groups immunized with the three vaccines that showed excellent bacterial clearance in the lungs exhibited no production of IFN-γ or other cytokines in the lung cells stimulated with antigens. However, in the spleen cells, the groups immunized with the antigens showed increased levels of IFN-γ, IL-2, and IL-17. In particular, the group immunized with Rv2299cD2D3-ESAT6-RpfED1-CWS (G6), which showed the best protective effect, did not produce any cytokines other than IFN-γ in the spleen cells. This suggests that the immune response had normalized in the lungs of the G5 and G6 groups, where bacterial clearance occurred, resulting in a markedly reduced immune response.

To date, no protein-based subunit vaccine/adjuvant combination has been reported to replace BCG in terms of vaccine efficacy. Therefore, protein-based subunit vaccines are being developed as BCG-prime booster vaccines. However, based on the results in FIGS. 16 to 19, the CWS-conjugated fusion protein antigens of the present disclosure surprisingly achieved complete bacterial clearance after just three vaccinations, demonstrating a far superior immune response compared to BCG. Accordingly, the fusion protein vaccine composition of the present disclosure is expected to be highly effective not only as a replacement for BCG but also as a BCG booster and therapeutic vaccine, as it induces an immune response that completely clears the bacteria.

### <EXAMPLE 4: Evaluation of Therapeutic Vaccine Efficacy of Conjugated Antigens of Rv2299c Domains, ESAT6, RpfE, and BCG Cell Wall Skeleton (BCG-CWS)>

Therapeutic vaccines aimed at improving tuberculosis treatment outcomes are being developed for purposes such as: i) preventing relapse after completing combination therapy, ii) enhancing the protective effect in cured patients, iii) reducing the duration of treatment or the number of drugs used after infection, and iv) preventing reactivation of latent tuberculosis. Accordingly, the therapeutic vaccine efficacy of the fusion protein antigens according to the present disclosure was evaluated.

### 4.1. Evaluation for Therapeutic Vaccine Potential of Rv2299c-ESAT6 Fusion Protein

To select therapeutic vaccines composed of highly active fusion proteins, the therapeutic vaccine efficacy of the previously developed vaccine candidate fusion protein Rv2299c-ESAT6 was primarily evaluated using a latent infection model. In this regard, an experiment scheme was configured as illustrated in FIG. 19A. First, *M. tuberculosis* was intratracheally administered to C57BL/6 mice, and after three weeks, when the bacterial load in the lungs reached its maximum, treatment with isoniazid (INH) and rifampin (RIF) was administered for four weeks. After the treatment stopped (Chemotherapy, C), no bacteria were initially detected in the lungs, but over time, bacterial regrowth occurred. At this point, vaccine candidates composed of the fusion proteins mixed with the adjuvants DDA/MPL were administered subcutaneously at weeks 4, 7, and 10 after the treatment ended, and the effect of suppressing bacterial regrowth was measured. The measurements are depicted in FIG. 19B. As seen in FIG. 19B, no bacteria were detected in the lungs of the control group injected with only DDA/MPL at week 7 post-infection, but by week 16, the bacterial count in the lungs had risen to the same level as the infection control group that had not been treated at all after infection. However, no bacteria were detected in the lungs of the group immunized with Rv2299c-ESAT6/DDA/MPL at week 7 post-infection, and by week 16, the bacterial count was significantly lower compared to the control group.

### 4.2. Evaluation for Vaccine Efficacy of Rv2299c-ESAT6-Rv3463 Fusion Protein

Based on the results shown in FIG. 19B, the vaccine efficacy of the Rv2299c-Rv3463 and Rv2299c-Rv3463-ESAT6 fusion proteins was evaluated. The experiment was conducted in the same manner as in FIG. 19A, and the bacterial counts in the lungs at weeks 12 and 20 post-infection are shown in FIG. 20B. As seen in FIG. 20B, at week 20 post-infection, the bacterial regrowth was better suppressed in the group immunized with Rv2299c-Rv3463-ESAT6 compared to the group immunized with Rv2299c-Rv3463. Then, using a different vaccine composition order, the fusion proteins Rv2299c-ESAT6 and Rv2299c-ESAT6-Rv3463 were prepared, and the same experiment was performed, with the results shown in FIG. 20C. As seen in FIG. 20C, at week 12 post-infection, bacterial regrowth was completely suppressed in the group immunized with Rv2299c-ESAT6-Rv3463, but by week 20, the difference between the groups immunized with Rv2299c-ESAT6 and Rv2299c-ESAT6-Rv3463 had disappeared. However, both vaccine groups still showed significant suppression of bacterial regrowth compared to the control group. Taken together, the results suggest that the fusion proteins Rv2299c-ESAT6, Rv2299c-ESAT6-Rv3463, and Rv2299c-Rv3463-ESAT6 exhibit excellent therapeutic vaccine efficacy, with Rv2299c-ESAT6-Rv3463 showing the best therapeutic vaccine efficacy at week 12 post-infection.

### 4.3. Evaluation for Vaccine Efficacy of Fusion Proteins of Various Active Domains and Rv2299c D2D3 Region

The Rv2299cD2D3-ESAT6 fusion protein, which is derived from the highly effective Rv2299c-ESAT6 vaccine but has a reduced molecular weight due to the removal of the Rv2299c D1 region, allows for the incorporation of multiple active domains. Accordingly, multiple fusion proteins were designed, cloned, and produced as recombinant proteins in *E. coli,* based on the Rv2299cD2D3-ESAT6 fusion protein, as shown in FIG. 21A. C57BL/6 mice infected with Mtb were treated and immunized as shown in FIG. 21B, and the bacterial counts in the lungs at week 20 post-infection are shown in FIG. 21C. Since the Rv2299c-ESAT6-Rv3463 fusion protein exhibited superior therapeutic vaccine efficacy as seen in FIG. 20, selection was made of vaccine candidates with better efficacy by using mice immunized with Rv2299cD2D3-ESAT6-Rv3463, in which the D1 domain of Rv2299c was removed from the fusion protein, as a reference. As seen in FIG. 21C, Rv2299cD2D3-ESAT6-Rv3463D2-Rv2882cD2-RpfED1-Rv2145cD2 (G7), Rv2299cD2D3-ESAT6-Rv3463D2-Rv2005cD3 (G8), and Rv2299cD2D3-ESAT6-Rv3463D2-Rv2005cD3-RpfED1 (G9) showed excellent vaccine efficacy.

Additionally, using Rv2299cD2D3-ESAT6 as the backbone, additional recombinant proteins were constructed as shown in FIG. 22A, and their vaccine efficacy was evaluated using the same method as shown in FIG. 22B, with the results presented in FIG. 22C. As shown in FIG. 22C, several fusion proteins demonstrated an inhibition of bacterial growth by approximately 0.5 log10. Interestingly, the basic backbone fusion protein, Rv2299cD2D3-ESAT6, showed the best vaccine efficacy, significantly inhibiting bacterial regrowth compared to the proteins with additional active domains fused to the backbone.

### 4.4. Evaluation for Vaccine Efficacy of Fusion Proteins Composed of Various Active Proteins or Domains

Multiple proteins were produced in the same manner as shown in FIG. 23A, and the therapeutic vaccine efficacy was analyzed as shown in FIG. 23B. Using Rv2299cD2D3-ESAT6-Rv3463 (G11) as a baseline, fusion proteins with better efficacy were identified. The vaccine efficacy of fusion proteins, including Rv2882c-Rv2005c-Rv3463, Rv2882c-Rv2005cD3-ESAT6-Rv3463, Rv2220-Rv2882cD2-Rv2005cD3-ESAT6-Rv3463D2, Rv0869c-Rv2882cD2-Rv2005cD3-ESAT6-Rv3463D2, Rv1605-Rv2882cD2-Rv2005cD3-ESAT6-Rv3463D2, Rv2220-ESAT6-Rv3463, Rv0869c-ESAT6-Rv2005cD3-Rv3463, and Rv1605-ESAT6-Rv2005cD3-Rv3463, was evaluated. As shown in FIG. 23C, the fusion protein Rv0869c-Rv2882cD2-Rv2005cD3-ESAT6-Rv3463D2 (G6) exhibited the best vaccine efficacy.

## Claims

1. A tuberculosis vaccine composition, comprising a fusion protein of an immune-active domain of Rv2299c, and ESAT6.

2. The tuberculosis vaccine composition of claim 1, wherein the fusion protein is a fusion protein of an immune-active domain of Rv2299c, composed of an amino acid sequence selected from the group consisting of SEQ ID NOS: 1 to 5, and ESAT6, composed of the amino acid sequence of SEQ ID NO: 6.

3. The tuberculosis vaccine composition of claim 1, wherein the fusion protein is a fusion protein of an immune-active domain of Rv2299c, composed of an amino acid sequence selected from the group consisting of SEQ ID NOS: 3 to 5, and ESAT6, composed of the amino acid sequence of SEQ ID NO: 6.

4. The tuberculosis vaccine composition of any one of claims 1 to 3, wherein the fusion protein has BCG-cell wall skeleton (BCG-CWS) conjugated to a terminus thereof.

5. The tuberculosis vaccine composition of any one of claims 1 to 3, wherein the tuberculosis vaccine composition is a vaccine for prevention or treatment of tuberculosis.

6. The tuberculosis vaccine composition of claim 4, wherein the tuberculosis vaccine composition is a vaccine for prevention or treatment of tuberculosis.

7. The tuberculosis vaccine composition of any one of claims 1 to 3, wherein the fusion protein further comprises, at a terminus thereof, at least one *M*. tuberculosis-derived antigen selected from the group consisting of RpfE, Rv3463, Rv2005c, Rv2882c, Rv2145c, Rv1605, Rv2220, and Rv0869c or an immune-active domain thereof.

8. The tuberculosis vaccine composition of any one of claims 1 to 3, wherein the fusion protein further comprises, at a terminus thereof, at least one *M*. tuberculosis-derived antigen or an immune-active domain thereof, the *M.* tuberculosis-derived antigen being any one selected from the group consisting of: RpfED1 including the amino acid of SEQ ID NO: 8; Rv3463 including the amino acid of SEQ ID NO: 19; Rv2005cD3-Rv3463 in which the amino acid sequences of SEQ ID NOS: 18 and 19 are sequentially linked and fused; Rv2882cD2-Rv2005cD3-Rv3463D2 in which the amino acid sequences of SEQ ID NOS: 12, 18, and 21 are sequentially linked and fused; Rv2005cD3-Rv1605-Rv3463D2 in which the amino acid sequences of SEQ ID NOS: 18, 22, and 21 are sequentially linked and fused; Rv3463D2-Rv2882cD2 in which the amino acid sequences of SEQ ID NOS: 21 and 12 are sequentially linked and fused; Rv3463D2-Rv2882cD2-Rv2145cD2 in which the amino acid sequences of SEQ ID NOS: 21, 12, and 15 are sequentially linked and fused; Rv3463D2-Rv2882cD2-RpfED1 in which the amino acid sequences of SEQ ID NOS: 21, 12, and 8 are sequentially linked and fused; Rv3463D2-Rv2882cD2-RpfED1-Rv2145cD2 in which the amino acid sequences of SEQ ID NOS: 21, 12, 8, and 15 are sequentially linked and fused; Rv3463D2-Rv2005cD3 in which the amino acid sequences of SEQ ID NOS: 21 and 18 are sequentially linked and fused; and Rv3463D2-Rv2005cD3-RpfED1 in which the amino acid sequences of SEQ ID NOS: 21, 18, and 8 are sequentially linked and fused.

9. The tuberculosis vaccine composition of claim 7, wherein the fusion protein has BCG-cell wall skeleton (BCG-CWS) conjugated to a terminus thereof.

10. The tuberculosis vaccine composition of claim 8, wherein the fusion protein has BCG-cell wall skeleton (BCG-CWS) conjugated to a terminus thereof.

11. The tuberculosis vaccine composition of claim 9, wherein the tuberculosis vaccine composition is a vaccine for prevention or treatment of tuberculosis.

12. The tuberculosis vaccine composition of claim 10, wherein the tuberculosis vaccine composition is a vaccine for prevention or treatment of tuberculosis.

13. A tuberculosis vaccine composition comprising a fusion protein as a *M. tuberculosis-derived* antigen or an immune-active domain thereof, the fusion protein being any one selected from the group consisting of: Rv2882c-Rv2005c-Rv3463 in which the amino acid sequences of SEQ ID NOS: 10, 16, and 19 are sequentially linked and fused; Rv2882c-Rv2005cD3-ESAT6-Rv3463 in which the amino acid sequences of SEQ ID NOS: 10, 16, 6, and 19 are sequentially linked and fused; Rv2220-Rv2882cD2-Rv2005cD3-ESAT6-Rv3463D2, Rv0869c-Rv2882cD2-Rv2005cD3-ESAT6-Rv3463D2 in which the amino acid sequences of SEQ ID NOS: 23, 12, 15, 6, and 21 are sequentially linked and fused; Rv1605-Rv2882cD2-Rv2005cD3-ESAT6-Rv3463D2 in which the amino acid sequences of SEQ ID NOS: 22, 12, 18, 6, and 21 are sequentially linked and fused; Rv2220-ESAT6-Rv3463 in which the amino acid sequences of SEQ ID NOS: 23, 6, 19, 6, and 21 are sequentially linked and fused; Rv0869c-ESAT6-Rv2005cD3-Rv3463 in which the amino acid sequences of SEQ ID NOS: 24, 6, 18, and 19 are sequentially linked and fused; and Rv1605-ESAT6-Rv2005cD3-Rv3463 in which the amino acid sequences of SEQ ID NOS: 22, 6, 18, and 19 are sequentially linked and fused.

14. The tuberculosis vaccine composition of claim 13, wherein the fusion protein has BCG-cell wall skeleton (BCG-CWS) conjugated to a terminus thereof.

15. The tuberculosis vaccine composition of claim 13 or 14, wherein the tuberculosis vaccine composition is a vaccine for prevention or treatment of tuberculosis.
